(19) Europäisches Patentamt / European Patent Office / Office européen des brevets

(11) **EP 3 189 853 A1**

# EUROPEAN PATENT APPLICATION
published in accordance with Art. 153(4) EPC

(43) Date of publication:
**12.07.2017 Bulletin 2017/28**

(21) Application number: **15829798.6**

(22) Date of filing: **04.08.2015**

(51) Int Cl.:
**A61K 39/39** (2006.01)     **A61K 45/00** (2006.01)
**A61P 37/02** (2006.01)     **A61P 43/00** (2006.01)

(86) International application number:
**PCT/JP2015/072103**

(87) International publication number:
**WO 2016/021601 (11.02.2016 Gazette 2016/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **04.08.2014 JP 2014159000**

(71) Applicant: **Nitto Denko Corporation**
**Ibaraki-shi, Osaka 567-8680 (JP)**

(72) Inventors:
• **SHISHIDO, Takuya**
  **Ibaraki-shi**
  **Osaka 567-8680 (JP)**

• **ASARI, Daisuke**
  **Ibaraki-shi**
  **Osaka 567-8680 (JP)**
• **MATSUSHITA, Kyohei**
  **Ibaraki-shi**
  **Osaka 567-8680 (JP)**
• **HORI, Mitsuhiko**
  **Ibaraki-shi**
  **Osaka 567-8680 (JP)**

(74) Representative: **Müller-Boré & Partner**
**Patentanwälte PartG mbB**
**Friedenheimer Brücke 21**
**80639 München (DE)**

(54) **IMMUNE-INDUCTION-PROMOTING COMPOSITION INCLUDING NUCLEAR RECEPTOR LIGAND, AND VACCINE PHARMACEUTICAL COMPOSITION**

(57)     The present invention aims to provide a composition for promoting immunity induction and a vaccine pharmaceutical composition which are universally usable for inducing immunity to various antigens and capable of exerting a high immunity inducing effect. The present invention relates to a composition for promoting immunity induction containing at least one nuclear receptor ligand and also relates to a vaccine pharmaceutical composition for inducing immunity containing an antigen and a composition for promoting immunity induction that is at least one nuclear receptor ligand.

**Description**

TECHNICAL FIELD

The present invention relates to a composition for promoting immunity induction containing a nuclear receptor ligand and a vaccine pharmaceutical composition for inducing immunity containing the composition for promoting immunity induction.

BACKGROUND ART

Common widely used vaccines are made from pathogens (e.g., microorganisms, viruses) or such pathogens whose toxicity is partially weakened or eliminated. The vaccines are administered to living bodies to induce immunity to prevent infectious diseases.

Dendritic cells after having engulfed viruses, microorganisms, or like foreign bodies migrate to lymph nodes and give naive T cells (Th0 cells) the information of the foreign bodies, thus inducing the differentiation of helper T cells. Through the interaction with dendritic cells, Th0 cells differentiate into type 1 helper T cells (Th1 cells), which are responsible for cellular immunity, and type 2 helper T cells (Th2 cells), which are responsible for humoral immunity (see Non-Patent Literature 1 and Non-Patent Literature 2, for example).

Many toll-like receptors (TLRs) are expressed in immunocompetent cells responsible for the innate immunity system, including dendritic cells. They are activated upon receiving a TLR ligand and promote the differentiation of helper T cells, thus activating immune reactions (see Non-Patent Literature 3, for example). For immunity activation, only the reaction routes via TLRs have been known, and other reaction routes have remained unclear.

It is known that immunity activation effects can be given by toxins such as cholera toxin or Escherichia coli heat-labile enterotoxin or fat/oil adjuvants that enhance the effects of immune reactions by slow-release of antigens. However, they have problems in terms of the balance between the safety and the efficacy (see Non-Patent Literature 4, for example).

CITATION LIST

- Non Patent Literature

Non-Patent Literature 1: Lipscomb MF. et al., Physiol Rev., 82, 97-130 (2002)
Non-Patent Literature 2: Zhou L. et al., Immunity, 30, 646-655 (2009)
Non-Patent Literature 3: Mazzoni A. et al., J Leukoc Biol., 75, 721-730 (2004)
Non-Patent Literature 4: Stevceva L. et al., Curr Pharm Des, 11, 801-811 (2005)

SUMMARY OF INVENTION

- Technical Problem

In view of the situation in the art, the present invention aims to provide a composition for promoting immunity induction and a vaccine pharmaceutical composition which are universally usable for inducing immunity to various antigens and effectively exert a biological defense effect such as cellular immunity or humoral immunity.

- Solution to Problem

The present inventors found out that externally stimulating a dendritic cell using a drug when the cell engulfs an antigen activates the innate immunity system as TLR ligands do, allowing effective induction of an immune reaction. The inventors made intensive studies focusing on the point that the control of dendritic cells, which are the starting point of immune reactions, allows effective induction of an antigen-specific immune reaction. They as a result found out that, surprisingly, antigen-specific immunity can be induced with a drug that acts on a nuclear receptor, which, completely unlike the TLR localized on the cell surface, exists within cells, particular within the nucleus, and is responsible for the information transduction to the inside of the nucleus and the transcriptional regulation. That is, the present invention found out that direct administration of a nuclear receptor ligand together with or separately from an antigen to the same site or different sites of a living body enables induction of an antigen-specific immune reaction through a reaction not involving a TLR stimulus by a TLR ligand.

Accordingly, the present invention is directed to a composition for promoting immunity induction containing a nuclear receptor ligand.

Preferably, the nuclear receptor ligand in the composition for promoting immunity induction of the present invention is at least one selected from the group consisting of a retinoid receptor agonist, a retinoid X receptor agonist, a thyroid hormone receptor agonist, and an estrogen receptor modulator.

Preferably, the nuclear receptor ligand in the composition for promoting immunity induction of the present invention is at least one selected from the group consisting of a retinoid receptor agonist, a thyroid hormone receptor agonist, and an estrogen receptor modulator and is for inducing humoral immunity.

Preferably the nuclear receptor ligand in the composition for promoting immunity induction of the present invention is at least one of a retinoid receptor agonist or a retinoid X receptor agonist and is for inducing cellular immunity.

Preferably, the composition for promoting immunity induction of the present invention further contains a helper peptide.

The present invention is also directed to a vaccine pharmaceutical composition containing an antigen for inducing immunity and the composition for promoting immunity induction.

Preferably, the vaccine pharmaceutical composition of the present invention is administered to a body surface.

Preferably, the vaccine pharmaceutical composition of the present invention is administered by intradermal injection, subcutaneous injection, or intramuscular injection.

In one embodiment of the present invention, the composition for promoting immunity induction and vaccine pharmaceutical composition containing an immunity induction promoter typified by a nuclear receptor ligand and a TLR ligand exert a higher humoral immunity inducing effect.

The present invention will be described in detail below.

The composition for promoting immunity induction and vaccine pharmaceutical composition of present invention are used for inducing antigen-specific immunity.

The antigen-specific immunity induction includes cellular immunity, which induces cytotoxic T-cells or the like, and humoral immunity, which promotes antibody production.

The cellular immunity inducing effect can be quantitatively determined by any method. Various methods have been developed. For example, the effect can be determined by an immunity induction experiment using an animal model for immunological evaluation and the ELISPOT assay (IFN-$\gamma$). The sample for the ELISPOT assay may be, for example, the spleen of the animal model for immunological evaluation.

The humoral immunity inducing effect may be quantitatively determined by any method. Various methods have been developed. For example, the effect can be determined by an immunity induction experiment using an animal model for immunological evaluation and ELISA (antigen-specific IgG antibody). The sample for ELISA may be, for example, blood of the animal model for immunological evaluation.

The composition for promoting immunity induction of the present invention contains an immunity induction promoter that is a nuclear receptor ligand. The vaccine pharmaceutical composition of the present invention contains an antigen and the composition for promoting immunity induction.

Owing to the inclusion of the antigen and the immunity induction promoter that is a nuclear receptor ligand, the vaccine pharmaceutical composition of the present invention can effectively induce an antigen-specific immune reaction.

As used herein, the term "antigen" means any substance that can induce an immune response. Any antigen can be used. Examples thereof include proteins and peptides. For transdermal administration, which requires antigens to permeate the skin, the antigen preferably has a small molecular weight. For example, a peptide containing about 8 to 12 amino acid residues can be used.

Any antigen can be used. Examples thereof include cancer antigen peptides, infectious pathogen-derived antigens, and infectious antigen peptides.

As used herein, the term "cancer" means abnormal expression of oncogene. Examples of the cancer include those associated with overexpression of a gene, such as hematological malignancy and solid cancer.

As used herein, the term "abnormal expression of a gene" means that the expression level of a gene in a cell significantly increases or decreases by, for example, at least two times or at least 4 times as compared to the expression level of another cell in the same tissue.

As used herein, the term "overexpression" means that the abnormal expression is an increase in the expression level. The gene expression level can be easily measured by any method well known in the art.

Examples of the oncogene include survivin gene, GPC3 gene, HER2/neu gene, MAGE3 gene, MAGE A1 gene, MAGE A3/A6 gene, MAGE A4 gene, MAGE12 gene, proteinase-3 gene, AFP gene, CA-125 gene, CD44 gene, CEA gene, c-Kit gene, c-met gene, c-myc gene, L-myc gene, COX2 gene, CyclinD1 gene, Cytokeratin-7 gene, Cytokeratin-19 gene, Cytokeratin-20 gene, E2F1 gene, E2F3 gene, EGFR gene, Gli1 gene, hCG$\beta$ gene, HIF-1$\alpha$ gene, HnRNP A2/B1 gene, hTERT gene, MDM gene, MDR-1 gene, MMP-2 gene, MMP-9 gene, Muc-1 gene, Muc-4 gene, Muc-7 gene, NSE gene, ProGRP gene, PSA gene, RCAS1 gene, SCC gene, thymoglobulin gene, VEGF-A gene, and VEGF-A gene.

Cancers associated with abnormal expression of the survivin gene include, but not limited to, malignant lymphoma, bladder cancer, lung cancer, and large bowel cancer. Cancers associated with abnormal expression of the GPC3 gene include, but no limited to, liver cancer, bile duct cancer, and stomach cancer. Cancers associated with abnormal expression of the HER2/neu gene include, but not limited to, breast cancer, stomach cancer, ovarian cancer, uterine cancer, bladder cancer, non-small cell lung cancer, and prostatic cancer. Cancers associated with abnormal expression of the MAGE3 gene include, but not limited to, melanoma, lung cancer, head and neck cancer, bladder cancer, stomach cancer, esophageal cancer, and liver cancer. Cancers associated with abnormal expression of the proteinase-3 gene include, but not limited to, acute myelocytic leukemia and pancreatic cancer.

As used herein, the term "cancer antigen" refers to a substance such as protein or peptide which is specifically expressed in tumor cells or cancer cells and capable of inducing a cellular immunity response.

As used herein, the term "cancer antigen peptide" refers to a partial peptide derived from a cancer antigen protein and capable of inducing a cellular immunity response. A cancer antigen peptide is usually generated by decomposition of a cancer antigen protein, an oncogene product, in a cancer cell and is presented on the surface of the cancer cell by MHC class I molecules.

The cancer antigen peptide may be an endogenous cancer antigen peptide isolated and purified from cancer cells, or a synthetic peptide having the same amino acid sequence as an endogenous cancer antigen peptide. Preferred specific examples of the cancer antigen peptide include survivin2B peptide, GPC3 peptide, HER2/neu_A24 peptide, MAGE3_A24 peptide, PR1 peptide, HER2/neu_A02 peptide, MAGE3_A02 peptide, HER2/neu_E75 peptide, MUC1 peptide, and altered peptides thereof.

As used herein, the term "survivin 2B peptide" means a peptide derived from survivin, an oncogene product, and having the sequence Ala Tyr Ala Cys Asn Thr Ser Thr Leu (SEQ ID No: 1).

As used herein, the term "GPC3 peptide" means a peptide derived from GPC3, an oncogene product, and having the sequence Glu Tyr Ile Leu Ser Leu Glu Glu Leu (SEQ ID No: 2).

As used herein, the term "HER2/neu_A24 peptide" means an HLA-A24-restricted peptide derived from HER2/neu, an oncogene product, and having the sequence Thr Tyr Leu Pro Thr Asn Ala Ser Leu (SEQ ID No: 3).

As used herein, the term "MAGE3_A24 peptide" means an HLA-A24-restricted peptide derived from MAGE3, an oncogene product, and having the sequence Ile Met Pro Lys Ala Gly Leu Leu Ile (SEQ ID No: 4).

As used herein, the term "PR1 peptide" means a peptide derived from proteinase-3, an oncogene product, and having the sequence Val Leu Gln Glu Leu Asn Val Thr Val (SEQ ID No: 5).

As used herein, the term "HER2/neu_A02 peptide" means a HLA-A02-restricted peptide derived from HER2/neu, an oncogene product, and having the sequence Lys Val Phe Gly Ser Leu Ala Phe Val (SEQ ID No: 6).

As used herein, the term "MAGE3_A02 peptide" means a HLA-A02-restricted peptide derived from MAGE3, an oncogene product, and having the sequence Lys Val Ala Glu Ile Val His Phe Leu(SEQ ID No: 7).

As used herein, the term "HER2/neu_E75 peptide" means a peptide derived from a product (HER2 protein) of the oncogene HER2/neu and having the sequence Lys Ile Phe Gly Ser Leu Ala Phe Leu (SEQ ID No: 8).

As used herein, the term "MUC1 peptide" means a peptide derived from MUC1 protein, which is a glycoprotein highly expressed on many cancer cells, and having the sequence Ser Thr Ala Pro Pro Val His Asn Val (SEQ ID No: 9).

As used herein, the term "altered peptide" means a peptide in which some or all of the amino acids are altered by substitution, modification, or the like.

The altered peptide is not limited. Examples thereof include (a) peptides having an amino acid sequence in which one or several amino acids (for example, 1, 2, 3, 4, or 5 amino acids) are substituted, deleted, or added; and (b) peptides having an amino acid sequence in which some or all of the amino acids (for example, 1, 2, 3, 4, 5, 6, 7, 8, 9, or 10 amino acids) are modified.

The amino acids of the altered peptide may be modified in any manner. Examples of the modification include acetylation, alkylation such as methylation, glycosylation, hydroxylation, carboxylation, aldehydation, phosphorylation, sulfonylation, formylation, aliphatic chain addition modification such as myristoylation, palmitoylation, or stearoylation, octanolyation, esterification, amidation, deamidation, disulfide bond formation modification such as cystine modification, glutathione modification, and thioglycolic acid modification, glycation, ubiquitination, succinimide formation, glutamylation, and prenylation.

The altered peptide may include substitution, deletion, or addition of one or more amino acids, and modification of one or more amino acids in combination.

The infectious pathogen-derived antigen refers to any substance that can be a target of an immune response generated by a subject living body. The infectious pathogen-derived antigen may be a substance that can be a target of an immune response (e.g., mature of an immunocompetent T-cell, increase in cytokine production, promotion of antibody production) when coming in contact with an immunocompetent cell.

An infectious disease can be addressed (for example, treated or prevented) by administrating the infectious disease-derived antigen and the composition for promoting immunity induction for inducing humoral immunity together to a subject using the vaccine pharmaceutical composition for inducing humoral immunity according to the present invention.

A cancer can be addressed (for example, treated or prevented) by administrating the cancer antigen and the composition for promoting immunity induction for inducing cellular immunity together to a subject using the vaccine pharmaceutical composition for inducing cellular immunity according to the present invention.

The infectious pathogen-derived antigen is not limited as long as it is an infectious pathogen or an antigen derived from an infectious pathogen.

The diseases caused by the infectious pathogens are not limited. Examples thereof include virus diseases caused by infection with viruses such as adenovirus (e.g., human adenovirus), herpesvirus (e.g., herpes simplex virus, varicella-zoster virus, cytomegalovirus, human herpesvirus, Kaposi sarcoma-associated herpesvirus), picornavirus (e.g., poliovirus, common cold virus, hepatitis A virus), poxvirus (e.g., smallpox virus, vaccinia virus, molluscum contagiosum virus), picornavirus (e.g., rhinovirus, enterovirus), orthomyxovirus (e.g., influenza virus), paramyxovirus (e.g., parainfluenza virus, mumps virus, measles virus, respiratory syncytial virus (RSV), Newcastle disease virus), parvovirus (e.g., adeno associated virus), togavirus (e.g., rubella virus), coronavirus (e.g., SARS coronavirus), hepadnavirus (e.g., hepatitis B virus), flavivirus (e.g., Japanese encephalitis virus, yellow fever virus, dengue virus, West Nile fever virus, St. Louis encephalitis virus, Murray Valley encephalitis virus, hepatitis C virus, hepatitis G virus), hepevirus (e.g., hepatitis E virus), papillomavirus (e.g., human papilloma virus), calicivirus (e.g., norovirus), rhabdovirus (e.g., rabies virus, vesicular stomatitis virus), filovirus (e.g., Ebola hemorrhagic fever virus), arenavirus (e.g., Lassa virus, hepatitis D virus), bunyavirus (e.g., California encephalitis virus, Rift Valley fever virus), reovirus (e.g., rotavirus), or retrovirus (e.g., human immunodeficiency virus (HIV), adult T-cell leukemia virus); bacterial diseases such as those caused by infection with a bacterium such as Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococci, Neisseria, Clostridium, Bacillus, Corynebacterium, Mycobacterium, Campyrobacter, Vibrio, Serratia, Providencia, Chromobacterium, Brucella, Yersinia, Haemophilus, or Bordetella; fungous diseases such as chlamydia, candidiasis, aspergillosis, histoplasmosis, and cryptococcal meningitis; malaria; pneumocystis carinii pneumonia; leishmaniasis; cryptosporidiosis; toxoplasmosis; and Trypanosoma infection.

As used herein, the term "infectious antigen peptide" means a partial peptide derived from an infectious pathogen-derived antigen protein and capable of inducing a cellular immunity response. The infectious pathogen-derived antigen is usually preferably IPEP87 peptide, HBVenv peptide, or an altered peptide thereof.

As used herein, the term "IPEP87 peptide" means a peptide derived from a hepatitis C virus (HCV) protein and having the sequence Asp Leu Met Gly Tyr Ile Pro Ala Val (SEQ ID No: 10).

As used herein, the term "HBVenv peptide" means a peptide derived from a hepatitis B virus (HBV) protein and having the sequence Trp Leu Ser Leu Leu Val Pro Phe Val (SEQ ID No: 11).

The above peptides can be in a free form or in the form of any pharmacologically acceptable salt.

Examples of the pharmacologically acceptable salt include acid salts (e.g., acetates, TFA salts, hydrochlorides, sulfates, phosphates, lactates, tartrates, maleates, fumarates, oxalates, hydrobromides, succinates, nitrates, malates, citrates, oleates, palmitates, propionates, formates, benzoates, picrates, benzenesulfonates, dodecylsulfates, methanesulfonates, p-toluenesulfonates, glutarates, amino acids salts), metal salts (e.g., alkali metal salts (e.g., sodium salts, potassium salts), alkaline earth metal salts (e.g., calcium salts, magnesium salts), aluminum salts), and amine salts (e.g., triethylamine salts, benzylamine salts, diethanolamine salts, t-butylamine salts, dicyclohexylamine salts, arginine salts, dimethylammonium salts, ammonium salts). Preferred are acetates and TFA salts.

The above peptides may be synthesized or produced, isolated, and purified by a well-known method.

In one preferred embodiment, immunity is effectively induced by administrating, together with or separately from an antigen, the composition for promoting immunity induction containing a nuclear receptor ligand, or by administrating the vaccine pharmaceutical composition containing an antigen and the composition for promoting immunity induction.

As used herein, the term "immunity induction promoter" means any substance that can improve the efficiency to induce immunity to an antigen administered with the substance, as compared to the efficiency obtained without the substance. The substance is not limited by the mechanism of promoting immunity induction, but the term means those specified herein.

In one preferred embodiment, immunity is effectively induced by administrating the vaccine pharmaceutical composition containing at least one nuclear receptor ligand selected from the group consisting of a retinoid receptor agonist, a retinoid X receptor agonist, a thyroid hormone receptor agonist, and an estrogen receptor modulator and an antigen.

As used herein, the term "nuclear receptor ligand" means a substance that acts on nuclear receptors or on protein related thereto. In one embodiment, the nuclear receptor ligand is an immunity induction promoter.

The nuclear receptor ligand may be a retinoid receptor ligand. The retinoid receptor ligand is preferably a retinoid receptor agonist. As used herein, the term "retinoid receptor agonist" means a substance that itself has a function to act on retinoid receptors. Examples of the retinoid receptor agonist include vitamin A, retinol palmitate, retinol acetate, retinol propionate, tazarotene, etretinate, acitretin, tretinoin, isotretinoin, alitretinoin, fenretinide, tamibarotene, palovarotene, and derivatives thereof, and pharmacologically acceptable salts thereof.

The nuclear receptor ligand may be a retinoid X receptor ligand. The retinoid X receptor ligand is preferably a retinoid X receptor agonist.

As used herein, the term "retinoid X receptor agonist" means a substance that itself has a function to act on retinoid X receptors. Examples of the retinoid X receptor agonist include bexaroten, and derivatives thereof, and pharmacologically acceptable salts thereof.

The nuclear receptor ligand may be a thyroid hormone receptor ligand. The thyroid hormone receptor ligand is preferably a thyroid hormone receptor agonist.

As used herein, the term "thyroid hormone receptor agonist" means a substance that itself has a function to act on thyroid hormone receptors. Examples of the thyroid hormone receptor agonist include tiratricol, levothyroxine, liothyronine, thyroid, liotrix, sobetirome, and derivatives thereof, and pharmacologically acceptable salts thereof.

The nuclear receptor ligand may be an estrogen receptor ligand. The estrogen receptor ligand is preferably an estrogen receptor modulator.

As used herein, the term "estrogen receptor modulator" means a substance that itself has a function to act on estrogen receptors to weakly activate the receptors and inhibit binding of estrogen. Examples of the estrogen receptor modulator include clomiphene, raloxifene, tamoxifen, toremifene, and derivatives thereof, and pharmacologically acceptable salts thereof.

The "salt" as used herein may be any organic acid salt or inorganic acid salt. The salt is preferably a pharmacologically acceptable salt.

As used herein, the "pharmacologically acceptable salt" means a salt that does not adversely affect the subject and does not eliminate pharmacological activity of the components of the vaccine pharmaceutical composition. Examples of such a salt include inorganic acid salts (e.g., hydrochlorides, phosphates), organic acid salts (e.g., acetates, phthalates, TFA salts, citrates), metal salts (e.g., alkali metal salts (e.g., sodium salts, potassium salts), alkaline earth metal salts (e.g., calcium salts, magnesium salts), aluminum salts), and amine salts (e.g., triethylamine salts, benzylamine salts, diethanolamine salts, t-butylamine salts, dicyclohexylamine salts, arginine salts, dimethylammonium salts, ammonium salts).

The amount of the immunity induction promoter that is a nuclear receptor ligand in the composition for promoting immunity induction of the present invention is not limited, but is preferably 0.0001 to 100% by weight, more preferably 0.001 to 80% by weight, still more preferably 0.01 to 50% by weight, most preferably 0.05 to 20% by weight based on the total weight of the composition.

The amount of the antigen in the vaccine pharmaceutical composition of the present invention is not limited, but is preferably 0.000001 to 50% by weight, more preferably 0.00001 to 20% by weight based on the total weight of the composition.

The amount of the immunity induction promoter that is a nuclear receptor ligand in the vaccine pharmaceutical composition of the present invention is not limited, but is preferably 0.001 to 10,000 parts by weight, more preferably 0.01 to 10,000 parts by weight based on 1 part by weight of the antigen.

If the amount of the immunity induction promoter is less than the lower limit, i.e., 0.001 parts by weight, the immunity inducing effect may be insufficient. If the amount of the immunity induction promoter is more than the upper limit, i.e., 10,000 parts by weight, safety issues may arise.

The vaccine pharmaceutical composition of the present invention may include, in addition to the above immunity induction promoter that is a nuclear receptor ligand, a second immunity induction promoter to the extent that does not impair the effect of the present invention. Any second immunity induction promoter can be used. Examples thereof include helper peptides.

The use of the above immunity induction promoter that is a nuclear receptor ligand in combination with the second immunity induction promoter allows more effective promotion of immunity induction.

When a helper peptide is used as the second immunity induction promoter, the helper peptide is preferably used as an immunostimulant for inducing cellular immunity.

As used herein, the term "helper peptide" means any peptide that activates helper T cells.

Examples of the second cellular immunity induction promoter that is a helper peptide include a helper peptide derived from tubercle bacillus, a helper peptide derived from measles virus, a helper peptide derived from hepatitis B virus, a helper peptide derived from hepatitis C virus, a helper peptide derived from Chlamydia trachomatis, a helper peptide derived from P. falciparum sporozoite, a helper peptide derived from keyhole limpet haemocyanin, helper peptide derived from tetanus toxin, a helper peptide derived from pertussis toxin, a helper peptide derived from diphtheria toxin, helper peptides derived from cancer cells (e.g., IMA-MMP-001 helper peptide, CEA-006 helper peptide, MMP-001 helper peptide, TGFBI-004 helper peptide, HER-2/neu(aa776-790) helper peptide, AE36 helper peptide, AE37 helper peptide, MET-005 helper peptide, and BIR-002 helper peptide), and universal helper analogs (e.g., PADRE), and altered peptides thereof. In particular, Peptide-25, altered Peptide-25, and PADRE are preferred.

As used herein, the term "PADRE" means a peptide of 13 amino acids having the sequence D-Ala Lys cyclohexyl-Ala Val Ala Ala Trp Thr Leu Lys Ala Ala D-Ala (SEQ ID No: 12).

In the composition for promoting immunity induction and vaccine pharmaceutical composition of the present invention, the amount of the second immunity induction promoter is not limited, but is preferably 0.001 to 500 parts by weight, more preferably 0.005 to 200 parts by weight, still more preferably 0.01 to 100 parts by weight based on 1 part by weight of the antigen. If the amount is less than the lower limit, i.e., 0.001 parts by weight, the immunity inducing effect may be insufficient. If the amount is more than the upper limit, i.e., 500 parts by weight, safety issues may arise.

The composition for promoting immunity induction and vaccine pharmaceutical composition of the present invention may contain additive(s), if necessary. The additives can be selected depending on, for example, the main components of the base, the compatibility with the antigen and the immunity induction promoter that is a nuclear receptor ligand, or intended administration regimen. Examples of the additives include isotonizing agents, antiseptics, antioxidants, resolvents, solubilizing agents, suspending agents, fillers, pH adjusters, stabilizers, absorption promoters, release-rate controlling agents, colorants, plasticizers, crosslinking agents, and adhesives. These additives may be used alone or in combination of two or more thereof.

Although the composition for promoting immunity induction and vaccine pharmaceutical composition of the present invention may be intradermally, subcutaneously, or intramuscularly administered, they are preferably administered to the body surface, more preferably transdermally or transmucosally administered. Accordingly, the vaccine pharmaceutical composition of the present invention may be a vaccine pharmaceutical composition for intradermal, subcutaneous, or intramuscular administration, but is preferably a vaccine pharmaceutical composition for transdermal administration or transmucosal administration.

The transdermal administration or transmucosal administration of the vaccine pharmaceutical composition of the present invention to a subject enables effective induction of antigen-specific humoral immunity.

As used herein, the term "subject" means any animal to which the vaccine pharmaceutical composition at a practical stage can be administered so as to induce an immune response. The term typically means mammals including human (e.g., mouse, rat, canine, feline, leporine, equine, bovine, ovine, porcine, caprine, simian, and chimpanzee). The subject is particularly preferably a human.

<Vaccine pharmaceutical composition for transmucosal administration>

The transmucosal administration may be, for example, sublingual administration, transnasal administration, buccal administration, rectal administration, and vaginal administration.

Examples of the dosage form of the vaccine pharmaceutical composition for transmucosal administration include semisolid formulations such as gels (jellies), creams, ointments, and plasters; solutions; solid formulations such as powders, fine granules, granules, films, tablets, and orally disintegrating tablets (freeze dry type); mucosal sprays such as aerosols, and inhalants. The categories, definition, characteristics, production processes, and the like of these compositions are well known in the art. See the Japanese Pharmacopoeia 16th edition, for example. The materials for these are not limited, and conventionally known materials can be used.

Preferred among the dosage forms are solutions and solid formulations (e.g., orally disintegrating tablets (freeze dry type), films).

Examples of the solvent used for the solutions include an appropriate amount of water, ethanol, glycerin, and propylene glycol. A solution can be prepared by dispersing or dissolving the ingredients (i.e., the antigen, the immunity induction promoter that is a nuclear receptor ligand, and if necessary, the second immunity induction promoter and the like) into the solvent.

Any base may be used for the gels (jellies). Examples thereof include hydrogel bases, such as carboxyvinyl polymers, gel bases, fat-free ointment, polyvinylpyrrolidone, polyvinyl alcohol, sodium polyacrylate, carboxymethylcellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxyvinyl polymers, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, Eudragit, casein, alkyl alginate, gelatin, and polyethylene glycol. A fluid gel or a gel with formability can be prepared by dissolving any of these bases into a solvent and adding the ingredients. The solvent is preferably water, but glycerin, propylene glycol, or the like can also be used.

Examples of the base used for the creams include water/oil-type bases such as hydrophilic ointment and vanishing cream; and oil/water-type bases such as hydrophilic Vaseline, purified lanolin, Aquahole, Eucerin, Neocerin, hydrous lanolin, cold cream, hydrophilic plastibase. A cream can be prepared by placing any of these bases into a fat/oil solvent or water and stirring the mixture at a high speed with, for example, a homogenizer.

Any base may be used for the films. Examples thereof include polyvinylpyrrolidone, polyvinyl alcohol, sodium polyacrylate, carboxymethylcellulose, starch, xanthan gum, karaya gum, sodium alginate, methylcellulose, carboxyvinyl polymers, agar, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxyvinyl polymers, tragacanth, gum arabic, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethylamino acetate, casein, and alkyl alginate. A film can be prepared by dissolving any of these bases in a polar organic solvent such as water or ethanol, adding the ingredients, applying the solution to form a thin film, and drying the film.

Any additives can be used for the powders, fine granules, granules, or tablets. Examples thereof include excipients such as lactose, corn starch, and crystalline cellulose, and binders such as hydroxypropylcellulose and gum arabic. Powder, fine granules, granules, and tablets can be prepared by adding these additives to an appropriate amount of solvent such as water or ethanol, adding the ingredients, mixing and stirring them, and then subjecting the resulting mixture to a combination of processes such as granulation, drying, and tablet compression. If necessary, a lubricant such as magnesium stearate and a coating agent such as hydroxypropylcellulose or sucrose may be added.

Any base may be used for the orally disintegrating tablets (freeze dry type). Examples thereof include polysaccharides such as gelatin and pullulan, and hydrogel bases such as hydroxypropylcellulose. Forming aids such as mannitol, trehalose, sorbitol, or glycine may also be used. An orally disintegrating tablet (freeze dry type) can be prepared by dissolving any of these bases and a forming aid in water, adding the ingredients, dispensing the resulting solution, and then freeze-drying the solution.

The content of the aerosol may be, for example, a solution, a highly fluidic gel, a cream, or fine powder such as a powdered drug. Dispersing the content as solid or liquid microparticles in a gas using a spray device enables effective administration to an administration site such as the oral mucosa or the nasal mucosa.

<Composition for promoting immunity induction for transmucosal administration>

The composition for promoting immunity induction for transmucosal administration according to the present invention allows, in mucosal administration of various nuclear receptor ligands to the subject, more effective exertion of immunity induced by various antigens administered together with or separately from the nuclear receptor ligands.

The administration route and the formulation of the composition for promoting immunity induction for transmucosal administration can be the same as those of the vaccine pharmaceutical composition for transmucosal administration. The formulation of the composition for promoting immunity induction for transmucosal administration can be prepared with the same materials as those used for preparing the formulation of the vaccine pharmaceutical composition for transmucosal administration.

<Vaccine pharmaceutical composition for transdermal administration>

Examples of the dosage form of the vaccine pharmaceutical composition for transdermal administration include solutions for external use such as liniments and lotions; sprays for external use such as aerosols; patches such as gels, tapes, poultices; ointments; plasters; and creams. The categories, definition, characteristics, production processes, and the like of these compositions are well known in the art. See the Japanese Pharmacopoeia 16th edition, for example. The materials for these are not limited, and conventionally known materials can be used.

Preferred among these dosage forms are creams and patches (e.g., tapes, poultices).

Examples of the base used for the liniments include water, ethanol, fatty oils, hard paraffin, soft paraffin, liquid paraffin, glycerin, paraffin oil, beeswax, metallic soap, mucilage, natural oils (e.g., almond oil, corn oil, peanut oil, castor oil, olive oil, and derivatives thereof (e.g., polyoxyl castor oil)), mutton tallow or derivatives thereof, and fatty acids and/or fatty acid esters (e.g., stearic acid, oleic acid, isopropyl myristate).

Lotion is a formulation containing the ingredients finely and homogenously dispersed in an aqueous liquid, and includes suspension lotion and emulsion lotion. The suspending agent may be, for example, gum arabic, sodium alginate, sodium carboxymethylcellulose, methylcellulose, or bentonite. The emulsifier may be, for example, sodium lauryl sulfate or sorbitan fatty acid ester.

Examples of the base used for the ointments include common hydrophobic bases such as fats and oils, waxes, and hydrocarbon compounds. Specific examples include mineral bases such as yellow Vaseline, white Vaseline, paraffin, liquid paraffin, plastibase, and silicone, and animal or vegetable bases such as beeswax and animal or vegetable oils and fats.

Examples of the base used for the creams include water/oil type bases such as hydrophilic ointment and

**EP 3 189 853 A1**

vanishing cream; and oil/water type bases such as hydrophilic Vaseline, purified lanolin, Aquahole, Eucerin, Neocerin, hydrous lanolin, cold cream, and hydrophilic plastibase.

Any base may be used for the gels. Examples thereof include hydrogel bases such as carboxyvinyl polymers, gel bases, fat-free ointment, polyvinylpyrrolidone, polyvinyl alcohol, sodium polyacrylate, carboxymethylcellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxyvinyl polymer, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethylamino acetate, casein, alkyl alginate, gelatin, and polyethylene glycol.

Examples of the base used for the poultices include gelatin, sodium carboxymethylcellulose, methylcellulose, sodium polyacrylate, kaolin, polyvinyl alcohol, polyvinylpyrrolidone, glycerin, propylene glycol, and water.

The tape preferably includes an adhesive layer containing ingredients (i.e., the antigen, the immunity induction promoter that is a nuclear receptor ligand, and if necessary, the second immunity induction promoter and the like) and a support that supports the adhesive layer. The tape may include a release liner that prevents exposure of the adhesive layer before use and can be easily removed at the time of use.

The adhesive forming the adhesive layer is not limited. Examples thereof include acrylic adhesives containing acrylic polymers; rubber adhesives containing rubber elastomers; silicone adhesives such as silicone rubber, dimethyl siloxane-based adhesives, and diphenyl siloxane-based adhesives; vinyl ether adhesives such as polyvinyl methyl ether, polyvinyl ethyl ether, and polyvinyl isobutyl ether; vinyl ester adhesives such as vinyl acetate-ethylene copolymer; and polyester adhesives containing a carboxylic acid component (e.g., dimethyl terephthalate, dimethyl isophthalate, dimethyl phthalate) and a polyalcohol component (e.g., ethylene glycol). Particularly preferred adhesives are acrylic adhesives, rubber adhesives, and silicone adhesives. For good antigen dispersibility/releasability, hydrophilic bases such as sodium polyacrylate are preferred.

The amount of the adhesive in the adhesive layer is not limited, but is preferably 10 to 90% by weight, more preferably 20 to 80% by weight in terms of solids based on the total weight of the adhesive layer.

The acrylic adhesive is preferably mainly composed of a polymer which contains alkyl (meth)acrylate as a first monomer.

Examples of the first monomer include (meth)alkyl acrylate having a C1-C18 linear, branched, or cyclic alkyl group. In particular, alkyl (meth)acrylate having a C4-C18 linear, branched, or cyclic alkyl group are preferred. Moreover, alkyl. (meth)acrylates having a C4-C8 linear, branched, or cyclic alkyl group (e.g., butyl, pentyl, hexyl, cyclohexyl, heptyl, octyl, or 2-ethylhexyl, preferably butyl, 2-ethylhexyl, or cyclohexyl, particularly preferably 2-ethylhexyl) are more preferred because the use of a monomer component that lowers the glass transition temperature of the polymer is more suitable for providing adhesiveness at normal temperature.

Specifically, butyl acrylate, 2-ethylhexyl acrylate, 2-ethylhexyl methacrylate, cyclohexyl acrylate, and cyclohexyl methacrylate are preferred as the first monomer, with 2-ethylhexyl acrylate being particularly preferred. These first monomers can be used alone or in combination of two or more thereof.

The first monomer may be copolymerized with a second monomer. Examples of the second monomer include monomers having a functional group that can be a crosslinking point when a crosslinking agent is used. Examples of such a functional group that can be involved with crosslinking reaction include a hydroxy group, a carboxy group, and a vinyl group, with a hydroxy group and a carboxy group being preferred.

Specific examples of the second monomer include hydroxyethyl (meth)acrylate, hydroxypropyl (meth)acrylate, N-hydroxyalkyl(meth)acrylamide, (meth)acrylic acid, itaconic acid, maleic acid, maleic anhydride, mesaconic acid, citraconic acid, and glutaconic acid. From the viewpoint of availability, acrylic acid, methacrylic acid, and hydroxyethyl acrylate (in particular, 2-hydroxyethyl acrylate) are preferred, with acrylic acid being most preferred. These second monomers may be used alone or in combination of two or more thereof.

The first and second monomer may be further copolymerized with a third monomer.

Examples of the third monomer include vinyl esters such as vinyl acetate and vinyl propionate; vinyl ethers such as methyl vinyl ether and ethyl vinyl ether; vinylamides such as N-vinyl-2-pyrrolidone and N-vinylcaprolactam; alkoxy (meth)acrylates such as methoxyethyl (meth)acrylate, ethoxyethyl (meth)acrylate, and tetrahydrofurfuryl (meth)acrylate; hydroxy group-containing monomers (as the third monomer, not as a crosslinking point) such as hydrox-ypropyl (meth)acrylate and $\alpha$-hydroxymethyl acrylate; (meth)acrylic acid derivatives having an amide group such as (meth)acrylamide, dimethyl(meth)acrylamide, N-butyl(meth)acrylamide, and N-methylol(meth)acrylamide; aminoalkyl (meth)acrylates such as aminoethyl (meth)acrylate, dimethylaminoethyl (meth)acrylate, and t-butylaminoethyl (meth)acrylate; alkoxyalkylene glycol (meth)acrylates such as methoxyethylene glycol (meth)acrylate, methoxydiethylene glycol (meth)acrylate, methoxypolyethylene glycol (meth)acrylate, and methoxypolypropylene glycol (meth)acrylate; (meth)acrylonitrile; monomers having a sulfonic acid such as styrenesulfonic acid, allylsulfonic acid, sulfopropyl (meth)acrylate, (meth)acryloyloxynaphthalenesulfonic acid, and acrylamidemethylsulfonic acid; and vinyl group-containing monomers such as vinylpiperidone, vinylpyrimidine, vinylpiperazine, vinylpyrrole, vinylimidazole, vinyloxazole, and vinylmorpholine. Preferred among these are vinyl esters, and vinylamides. A preferred vinyl ester is vinyl acetate, and a preferred vinylamide is N-vinyl-2-pyrrolidone. These third monomers can be use alone or in combination of two or more thereof.

When the acrylic adhesive is a copolymer of alkyl (meth)acrylate (first monomer) and a vinyl monomer (second monomer) having a functional group that can be involved with crosslinking reaction, the weight ratio between the alkyl (meth)acrylate and the vinyl monomer having a functional group that can be involved with crosslinking reaction is preferably 99 to 85:1 to 15, more preferably 99 to 90:1 to 10.

When the acrylic adhesive is a copolymer of alkyl (meth)acrylate (first monomer), a vinyl monomer (second monomer) having a functional group that can be involved with crosslinking reaction, and a different monomer (third monomer), the weight ratio between the alkyl (meth)acrylate, the vinyl monomer having a functional group that can be involved with crosslinking reaction, and the different monomer is preferably 40 to 94:1 to 15:5 to 50, more preferably 50 to 89:1 to 10:10 to 40.

The polymerization reaction is not limited, and may be performed by a known method per se. For example, the above monomers are reacted in the presence of a polymerization initiator (e.g., benzoyl peroxide, azobisisobutyronitrile) in a solvent (e.g., ethyl acetate) at 50°C to 70°C for 5 to 48 hours.

The acrylic adhesives more preferably contains any of 2-ethylhexyl acrylate/acrylic acid/N-vinyl-2-pyrrolidone copolymer, 2-ethylhexyl acrylate/N-(2-hydroxyethyl)acrylamide/N-vinyl-2-pyrrolidone copolymer, 2-ethylhexyl acrylate/2-hydroxyethyl acrylate/vinyl acetate copolymer, and 2-ethylhexyl acrylate/acrylic acid copolymer, particularly preferably contains 2-ethylhexyl acrylate/acrylic acid/N-vinyl-2-pyrrolidone copolymer.

The acrylic adhesives may be subjected to physical crosslinking by radiation such as UV irradiation or electron beam irradiation, or may be subjected to chemical crosslinking using crosslinking agents such as an isocyanate compound (e.g., trifunctional isocyanate), an organic peroxide, an organic metal salt, a metal alcoholate, a metal chelate compound, or a multifunctional compound (e.g., a multifunctional external crosslinking agent, a multifunctional monomer for internal crosslinking such as di(meth)acrylate).

The rubber elastomer forming the rubber adhesive is not limited. Examples thereof include polyisobutylene-polybutene elastomer, styrene-diene-styrene block copolymer, styrene-butadiene elastomer, nitrile elastomer, chloroprene elastomer, vinylpyridine elastomer, polyisobutylene elastomer, butyl elastomer, and isoprene-isobutylene elastomer. In particular, from the viewpoint of the solubility to the ingredients and the adhesiveness to the skin, preferred elastomers are polyisobutylene (PIB) and styrene-diene-styrene block copolymers (e.g., styrene-butadiene-styrene block copolymer (SBS), styrene-isoprene-styrene block copolymer (SIS)). These rubber elastomers may be used alone or in combination of two or more thereof.

In order to obtain suitable adhesion and suitable solubility to the ingredients, the rubber adhesive may contain a mixture of rubber elastomers that are the same as or different in the components and different in the average molecular weight. For example, preferred is a mixture of a high-molecular-weight polyisobutylene with an average molecular weight of 150,000 to 5,500,000, a medium-molecular-weight polyisobutylene with an average molecular weight of 10,000 to 150,000, and/or a low-molecular-weight polyisobutylene with an average molecular weight of 500 to 4,000. In the mixture, the amount of the high-molecular-weight polyisobutylene is 10 to 80% by weight, preferably 20 to 70% by weight based on the total amount of the polyisobutylenes. The amount of the medium-molecular weight polyisobutylene is 0 to 90% by weight, preferably 10 to 80% by weight based on the total amount of the polyisobutylenes. The amount of the low-molecular-weight polyisobutylene is 0 to 80% by weight, preferably 10 to 60% by weight based on the total amount of the polyisobutylenes.

The "average molecular weight" as used herein means the viscosity average molecular weight calculated by Flory's viscosity equation. The average molecular weight is determined by calculating Staudinger index ($J_0$) from the flow time at 20°C of the capillary 1 of an Ubbelohde viscometer by Schulz-Blaschke equation and calculating the viscosity average molecular weight using the $J_0$ value according to the formula below.

$$J_0 = \eta_{sp}/c\,(1 + 0.31\,\eta_{sp}) \quad \text{(Schulz-Blaschke equation)}$$

$$\eta_{sp} = t/t_0 - 1$$

t: Flow time of solution (according to Hagenbach-couette correction formula)
to: Flow time of solvent (according to Hagenbach-couette correction formula)
c: Concentration of solution (g/cm$^3$)

$$J_0 = 3.06 \times 10^{-2} \overline{M_V}^{0.65}$$

$\overline{M_V}$ : Viscosity average molecular weight

In order to provide suitable tackiness, the rubber adhesive may contain a tackifier, such as rosin resin, polyterpene resin, coumarone-indene resin, petroleum resin, terpene-phenol resin, xylene resin, or alicyclic saturated hydrocarbon resin. These tackifiers may be used alone or in combination of two or more thereof.

The tackifier content is preferably 50% by weight or less, preferably 5 to 40% by weight based on the total weight of the rubber adhesive.

Examples of the silicone adhesives include polyorganosiloxane adhesives, polydimethylsiloxane adhesives, and polydimethyldiphenyl-siloxane adhesives. In particular, commercially available silicone adhesives, such as BIO PSA from Dow Corning Corporation, are preferred.

The adhesive layer may further contain a skin permeation enhancer.

As used herein, the term "skin permeation enhancer" refers to a substance that can improve the efficiency at which a transdermally administered antigen permeates the skin.

The skin permeation enhancer is preferably liquid, that is, fluidic, at room temperature (25°C). When a mixture of two or more skin permeation enhancers is used, the resulting mixture is preferably liquid at room temperature (25°C) and has a skin permeation promoting effect. Such an organic liquid component is preferably a hydrophobic liquid component from the viewpoint of the compatibility in the adhesive layer.

Examples of the skin permeation enhancer include higher alcohols, fatty acid esters, and polyalcohol fatty acid esters.

Preferred among the higher alcohols are C8-C18 higher alcohols, and more preferred are C8-C14 higher alcohols. Preferred among the fatty acid esters are fatty acid esters of C8-C18 fatty acids with C1-C18 monohydric alcohols, and more preferred are fatty acid esters of C12-C16 fatty acids with C1-C18 monohydric alcohols. In particular, fatty acid esters are preferred, and isopropyl myristate, isopropyl palmitate, and diethyl sebacate are particularly preferred.

Specific examples of the skin permeation enhancer include higher alcohols such as oleyl alcohol and octyl dodecanol; polyalcohols such as glycerin, ethylene glycol, and polypropylene glycol; higher fatty acids such as oleic acid and caplyric acid; fatty acid esters such as isopropyl myristate, isopropyl palmitate, and ethyl oleate; polybasic acid esters such as diethyl sebacate and diisopropyl adipate; polyalcohol fatty acid esters such as diglyceryl triisostearate, sorbitan monooleate, propylene glycol dicaprylate, polyethylene glycol monolaurate, and polyoxyethyelene sorbitol tetraoleate; polyoxyethylene alkyl ether such as polyoxyethylene lauryl ether; hydrocarbons such as squalane and liquid paraffin; vegetable oils such as olive oil and castor oil; silicone oil; pyrrolidones such as N-methylpyrrolidone and N-dodecylpyrrolidone; and sulfoxides such as decylmethylsulfoxide. These can be used alone or in combination of two or more thereof.

When the rubber or acrylic adhesive is used, the skin permeation enhancer may be polyvinylpyrrolidone, crospovidone, polypropylene glycol, polyvinyl alcohol, carboxyvinyl polymer, hydroxypropylcellulose, or a mixture thereof. In particular, polyvinylpyrrolidone, crospovidone, and polypropylene glycol are preferred.

The amount of the skin permeation enhancer in the adhesive layer is not limited, but is preferably 0.1 to 70% by weight, more preferably 1 to 65% by weight, still more preferably 5 to 60% by weight based on the total weight of the adhesive layer. When the amount of the skin permeation enhancer is 0.1% by weight or more, a high skin permeation promoting effect can be obtained. When the amount of the skin permeation enhancer is 70% by weight or less, a high skin permeation promoting effect can be obtained while reduction in the adhesion and cohesion of the entire adhesive layer can be suppressed.

The thickness of the adhesive layer is not limited, but is preferably 10 to 1,000 μm. With the thickness being in the range, the adhesive layer can easily contain the ingredients in effective amounts, easily exhibit sufficient adhesion, and be easily formed.

The support is not limited. Preferably, the support is one substantially impermeable to the ingredients, that is, one that prevents reduction in the amounts of the antigen, the immunity induction promoter that is a nuclear ligand, and if necessary the second immunity induction promoter in the adhesive layer by not allowing them to pass through the support and escape from the back side.

The support may be, for example, a single film containing polyester, polyamide, polyvinylidene chloride, polyethylene, polypropylene, polyvinyl chloride, ethylene-ethyl acrylate copolymer, polytetrafluoroethylene, ionomer resin, or metallic foil or may be a laminated film containing such films. In particular, in order to improve adhesiveness (anchoring properties) between the support and the adhesive layer, the support is preferably a laminated film including a non-porous plastic film and a porous film each containing any of the above materials. In this case, the adhesive layer is preferably formed on the porous film-side.

The porous film may be any porous film that improves the anchoring properties between the support and the adhesive layer. Examples thereof include paper, woven fabrics, nonwoven fabrics, knitted fabrics, and mechanically perforated sheets. Preferred among these are paper, woven fabrics, and nonwoven fabrics from the viewpoint of the handleability. The porous film preferably has a thickness of 1 to 200 $\mu$m from the viewpoint of improving the anchoring properties and also from the viewpoint of the flexibility and attachment operability of the tape. When the porous film is a woven fabric or a nonwoven fabric, the weight per unit area thereof is preferably 5 to 30 g/m$^2$, more preferably 6 to 15 g/m$^2$.

The support is preferably a laminated film including a polyester film (preferably, polyethylene terephthalate film) with a thickness of 1.5 to 6 $\mu$m and a polyester (preferably, polyethylene terephthalate) nonwoven fabric with a weight per unit area of 6 to 15 g/m$^2$.

Any release liner can be used as long as it is release-treated and can be peeled with sufficiently light force. For example, the release liner may be a film formed of polyester, polyvinyl chloride, polyvinylidene chloride, or polyethylene terephthalate, paper such as woodfree paper or glassine, or a laminated film including woodfree paper or glassine and a polyolefin, which are release-treated by applying silicone resin, fluororesin, or the like to the side to be in contact with the adhesive layer.

The release liner has a thickness of preferably 10 to 200 $\mu$m, more preferably 25 to 100 $\mu$m. From the viewpoint of barrier properties and the price, the release liner is preferably formed of a polyester (in particular, polyethylene terephthalate) resin. In this case, from the viewpoint of the handleability, the thickness of the release liner is preferably about 25 to 100 $\mu$m.

<Composition for promoting immunity induction for transdermal administration>

The composition for promoting immunity induction for transdermal administration according to the present invention allows, in transdermal administration of various nuclear receptor ligands to the subject, more effective exertion of immunity induced by various antigens administered together with or separately from the nuclear receptor ligands.

The formulation of the composition for promoting immunity induction for transdermal administration may be the same as that of the vaccine pharmaceutical composition for transdermal administration. The formulation of the composition for promoting immunity induction for transdermal administration can be prepared with the same materials as those used for preparing the vaccine pharmaceutical composition for transdermal administration.

<Vaccine pharmaceutical composition for intradermal, subcutaneous, or intramuscular administration>

The dosage form of the vaccine pharmaceutical composition for intradermal, subcutaneous, or intramuscular administration is one that has a certain degree of fluidity that allows administration by injection, such as a solution, an aqueous or hydrophobic suspension, or a cream. The categories, definition, characteristics, production processes, and the like of these compositions are well known in the art. See the Japanese Pharmacopoeia 16th edition, for example.

The materials for these are not limited, and conventionally known materials can be used.

The solvent for the solution may be, for example, an appropriate amount water or saline, ethanol, glycerin, or propylene glycol. A solution can be prepared by dispersing or dissolving the ingredients into the solvent.

Any base may be used for the aqueous suspension include hydrogel bases, such as carboxyvinyl polymers, gel bases, fat-free ointment, polyvinylpyrrolidone, polyvinyl alcohol, sodium polyacrylate, carboxymethylcellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxyvinyl polymers, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, Eudragit, casein, alkyl alginate, gelatin, and polyethylene glycol. A fluidic suspension can be prepared by dissolving any of these bases into a solvent and adding the ingredients. The solvent is preferably saline, but glycerin, propylene glycol, or the like can also be used.

Examples of the base for hydrophobic suspension include water/oil-type bases such as hydrophilic ointment and vanishing cream; and oil/water-type bases such as hydrophilic Vaseline, purified lanolin, Aquahole, Eucerin, Neocerin, hydrous lanolin, cold cream, and hydrophilic plastibase. A fat/oil suspension can be prepared by placing any of these bases into a fat/oil solvent or water, stirring the mixture at a high speed with, for example, a homogenizer, and adding the ingredients.

<Composition for promoting immunity induction for intradermal, subcutaneous, or intramuscular administration>

The composition for promoting immunity induction for intradermal, subcutaneous, or intramuscular administration according to the present invention allows, in intradermal, subcutaneous, or intramuscular administration of various nuclear receptor ligands to the subject, more effective exertion of immunity induced by various antigens administered together with or separately from the nuclear receptor ligands.

The formulation of the composition for promoting immunity induction for intradermal, subcutaneous, or intramuscular administration may be the same as that of the vaccine pharmaceutical composition for intradermal, subcutaneous, or intramuscular administration. The formulation of the composition for promoting immunity induction for intradermal, subcutaneous, or intramuscular administration can be prepared with the same materials as those used for preparing the formulation of the vaccine pharmaceutical composition for intradermal, subcutaneous, or intramuscular administration.

When the vaccine pharmaceutical composition of the present invention is administered to the subject, the therapeutically effective amount thereof may widely vary depending on severity of the disease, age and relative health of the subject and other known factors. Generally, satisfactory results can be obtained at a dose of about 0.1 $\mu$g to 1 g/kg body weight per day. The immunity induction promoter that is a nuclear receptor ligand is simultaneously or sequentially, preferably simultaneously, administered with the antigen.

When the composition for promoting immunity induction of the present invention is administered to the subject, and when the vaccine pharmaceutical composition containing the composition for promoting immunity induction is administered to the subject, the therapeutically effective amount of the immunity induction promoter that is a nuclear receptor ligand may widely vary depending on factors such as the specific nuclear receptor ligand used or the presence or absence of other immunity induction promoter(s). Generally, satisfactory results can be obtained at a dose of about 0.01 $\mu$g to 1 g/kg body weight per day.

The daily dose may be administered in one time, or may be divided into multiple doses (i.e., two or more doses, for example, 2, 3, 4, or 5 doses). The period of continuous administration per dose is preferably appropriately selected in the range from 1 minute to 7 days. The administration interval is preferably appropriately selected from once daily to yearly (e.g., once a day, once every two days, once every three days, once a week, once every two weeks, once a month, once every three months, once every six months, once a year) or longer administration intervals, depending on, for example, the condition of the patient, the severity of the disease, or whether it is for therapeutic purposes or preventive purposes. Generally, for therapeutic purposes for patients actually having a severe disease, the vaccine pharmaceutical composition of the present invention is preferably administered with a higher frequency and/or with a higher dose. For preventive purposes for patients not having a disease, the vaccine pharmaceutical composition of the present invention is preferably administered with a lower frequency and/or with a lower dose.

- Advantageous Effects of Invention

The composition for promoting immunity induction and vaccine pharmaceutical composition of the present invention can be non-invasively administered (e.g., transdermally or transmucosally administered) to the body surface or minimally invasively administered to the skin surface (e.g., to the surface of the skin after a corneum removal treatment such as tape stripping or after a corneum perforation treatment such as a microneedle treatment or electroporation), therefore leading to excellent compliance. In other words, problems concerned with QOL of patients, such as pain, fear, injection scars with subsequent cicatrization, or regular hospital visits putting a burden to patients in the case of repetitive administrations, can be reduced. Moreover, as the compositions are easy to administer, patients can administer the compositions by themselves, reducing the risk of infections of health care workers via needle stick injury. Furthermore, medical wastes requiring specific waste treatment, such as injection needles, are not generated.

The composition for promoting immunity induction and vaccine pharmaceutical composition of the present invention in a patch form, such as a tape or a poultice, are advantageous in that they enables secure administration of a predetermined dose and the control of the drug releasing rate at any rate, and that they do not attach to unintended sites when administered. The compositions in a patch form are advantageous also in that since a patch is easily removed, patients can immediately stop the administration by themselves by removing the patch from the application site if any adverse effect occurs.

Administration of the composition for promoting immunity induction of the present invention or the vaccine pharmaceutical composition of the present invention provides a significantly improved antibody production inducing effect as compared to administration of an antigen alone.

BRIEF DESCRIPTION OF DRAWINGS

EP 3 189 853 A1

Fig. 1 is a graph showing evaluation results of the number of IFN-γ-producing cell spots in mouse spleen cells after transdermal administration of creams for transdermal administration obtained in Examples 1 to 8 and Comparative Examples 1 and 2.

Fig. 2 is a graph showing evaluation results of the number of IFN-γ-producing cell spots in mouse spleen cells after transdermal administration of tapes for transdermal administration obtained in Examples 9 to 20 and Comparative Examples 3 to 8.

Fig. 3 is a graph showing an OVA-specific IgG antibody titer in mouse serum after transnasal administration of solutions for transnasal administration obtained in Examples 21 to 28 and Comparative Example 9.

Fig. 4 is a graph showing an OVA-specific IgG antibody titer in mouse serum after sublingual administration of solutions for sublingual administration obtained in Examples 29 to 36 and Comparative Example 10.

Fig. 5 is a graph showing an OVA-specific IgG antibody titer in mouse serum after sublingual administration of solid formulations for sublingual administration obtained in Examples 37 to 52 and Comparative Examples 11 and 12.

Fig. 6 is a graph showing an OVA-specific IgG antibody titer in mouse serum after subcutaneous administration of solutions for subcutaneous administration obtained in Examples 53 to 56 and Comparative Example 13.

Fig. 7 is a graph showing an OVA-specific IgG antibody titer in mouse serum after transdermal administration of creams obtained in Examples 57 to 60 and Comparative Example 14.

DESCRIPTION OF EMBODIMENTS

The present invention will be specifically described in further detail below. The present invention, however, is not limited to these examples.

(Examples 1 to 8, Comparative Examples 1 and 2)

(Preparation of cream for transdermal administration)

A cream for transdermal administration was prepared according to the formulation shown in Table 1 below. Specifically, 5% by weight of an antigen peptide, 3% by weight of a nuclear receptor ligand, and if necessary 1 part by weight of a helper peptide and 15% by weight of dimethylsulfoxide (DMSO) (listed below) were blended in the amounts shown in Table 1. A base (base cream) was then added to achieve a total amount of 100% by weight, followed by mixing to provide a cream for transdermal administration. The base cream was prepared by blending and mixing materials according to the formulation in Table 14. White Vaseline, sorbitan monostearate, isostearic acid, benzyl alcohol, stearyl alcohol, polysorbate 60, concentrated glycerin, dimethylsulfoxide (DMSO) were purchased from Wako Pure Chemical Industries, Ltd. Cetanol was purchased from Tokyo Chemical Industry Co., Ltd.

A composite base was prepared by bonding a PET film/PET nonwoven fabric laminate (area: 0.7 cm$^2$) to the center portion of an adhesive tape for attachment such that the PET film-side faced the tape. The cream for transdermal administration in an amount of 4 mg was applied to the nonwoven fabric portion of the composite base to provide a sample for an immunity test.

(Nuclear receptor ligand)

Tretinoin (all-trans-retinoic acid, Wako Pure Chemical Industries, Ltd.)
Isotretinoin (13-cis-retinoic acid, Sigma-Aldrich)
Alitretinoin (9-cis-retinoic acid, Wako Pure Chemical Industries, Ltd.)
Bexarotene (Sigma-Aldrich)

(Antigen peptide)

OVAp (OVA peptide, peptide of 8 amino acids having the sequence Ser Ile Ile Asn Phe Glu Lys Leu (SEQ ID No: 13))

(Helper peptide)

PADRE

<Evaluation 1>

Each of the creams for transdermal administration obtained in the examples and comparative examples were subjected to the following evaluations.

(Evaluation of cellular immunity inducing effect)

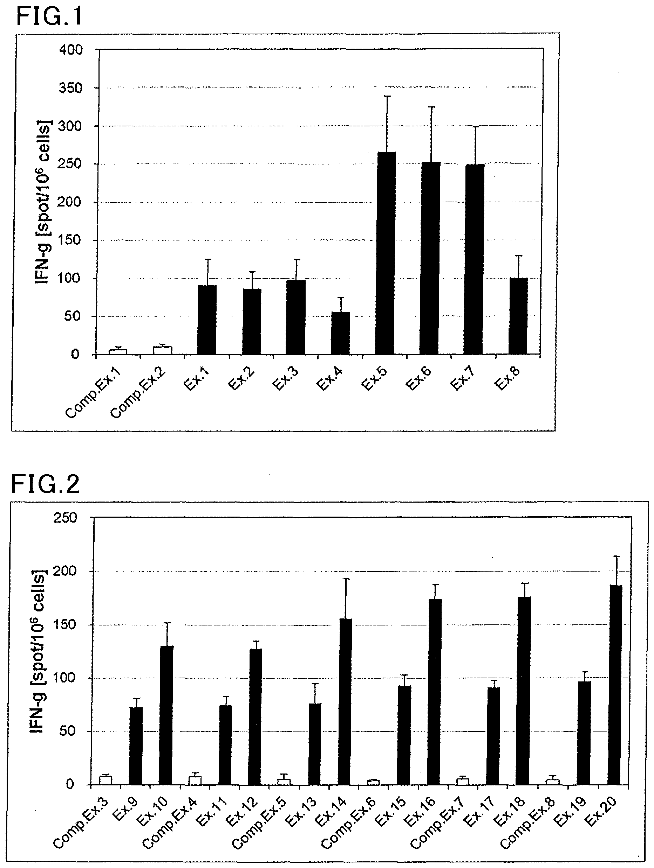

A mouse immunity test using an animal model for immunological evaluation was performed with the cream for transdermal administration by the following procedure. Thereafter, the level of induction of antigen-specific cellular immunity was evaluated by the ELISPOT assay. The results are shown in Fig. 1.

(1) Animal model for immunological evaluation

The "animal model for immunological evaluation" herein means an animal model for evaluating immunity induction properties of a vaccine pharmaceutical composition (here, the cream for transdermal administration). Specifically, the term means an animal model for evaluating the level of induction of cellular immunity of the cream for transdermal administration.
The animal model for immunological evaluation used was an animal in which the induction of cellular immunity by the antigen in the cream for transdermal administration can be evaluated, in view of the compatibility of the antigen in the cream for transdermal administration with MHC class 1 molecules of the animal.
Specifically, for vaccine pharmaceutical compositions containing HLA-A*24 type MHC-restricted class I peptide as the antigen, evaluation was performed using BALB/c mice. For vaccine pharmaceutical compositions containing HLA-A*02 type MHC-restricted peptide as the antigen, evaluation was performed using genetically engineered mice in which the induction of cellular immunity by HLA-A*02 type MHC-restricted peptide could be evaluated. For vaccine pharmaceutical compositions containing other HLA-type MHC-restricted peptides, evaluation was performed using animals in which the induction of cellular immunity by the HLA-type MHC-restricted peptides could be evaluated.

(2) Mouse immunity test of cream for transdermal administration

The back of the mouse shown in Table 1 below was shaved. After a rearing period for recovery from damage from the shaving, 4 mg of the cream for transdermal administration was administered to the skin of the back of the mouse for 24 hours and removed. The mouse was reared for six days. Six days after the administration, the spleen was taken out, and a spleen cell suspension was prepared. Spleen cells ($1 \times 10^6$ cells/well) and the antigen peptide (100 $\mu$M) together with culture medium were put into wells of an ELISPOT plate on which anti-mouse IFN-$\gamma$ antibody was immobilized. The spleen cells were co-cultured with the antigen at 37°C and 5% $CO_2$ for 20 hours. The number of IFN-$\gamma$-producing cell spots was evaluated by the ELISPOT assay. The number of IFN-$\gamma$-producing spots is shown as "Immunity result" in Table 1.

[Table 1]

| No. | Administration route | Dosage form | Antigen | | Nuclear receptor ligand | | Helper peptide | | Immunological evaluation mouse | Immunity result |
|---|---|---|---|---|---|---|---|---|---|---|
| | | | Name | Amount [Parts by weight] | Name | Amount [Parts by weight] | Name | Amount [Parts by weight] | | |
| Comp. Ex. 1 | Transdermal | Cream | OVAp | 5 | - | - | PADRE | - | C57BL/6 | 6.0 |
| Comp. Ex. 2 | Transdermal | Cream | OVAp | 5 | - | - | PADRE | 1 | C57BL/6 | 9.6 |
| Ex. 1 | Transdermal | Cream | OVAp | 5 | Tretinoin | 3 | PADRE | - | C57BL/6 | 90.4 |
| Ex. 2 | Transdermal | Cream | OVAp | 5 | Isotretinoin | 3 | PADRE | - | C57BL/6 | 85.6 |
| Ex. 3 | Transdermal | Cream | OVAp | 5 | Alitretinoin | 3 | PADRE | - | C57BL/6 | 97.5 |
| Ex. 4 | Transdermal | Cream | OVAp | 5 | Bexarotene | 3 | PADRE | - | C57BL/6 | 55.4 |
| Ex. 5 | Transdermal | Cream | OVAp | 5 | Tretinoin | 3 | PADRE | 1 | C57BL/6 | 264.9 |
| Ex. 6 | Transdermal | Cream | OVAp | 5 | Isotretinoin | 3 | PADRE | 1 | C57BL/6 | 252.0 |
| Ex. 7 | Transdermal | Cream | OVAp | 5 | Alitretinoin | 3 | PADRE | 1 | C57BL/6 | 248.3 |
| Ex. 8 | Transdermal | Cream | OVAp | 5 | Bexarotene | 3 | PADRE | 1 | C57BL/6 | 99.8 |

(Examples 9 to 20, Comparative Examples 3 to 8)

(Preparation of tape for transdermal administration)

A tape for transdermal administration was prepared according to the formulation shown in Table 2 below. Specifically, an antigen peptide, a nuclear receptor ligand, and a helper peptide (listed below) were mixed in amounts shown in Table 2. An adhesive base and an organic solvent (ethyl acetate) shown in Table 2 were then added so that the total amount of the components and the adhesive base after drying the organic solvent was 100% by weight. This was followed by mixing to provide an adhesive solution. The resulting adhesive solution was spread onto a release liner such that the thickness after drying was about 80 $\mu$m. The organic solvent was removed by drying, whereby an adhesive layer was formed. The release liner was a polyethylene terephthalate (PET) liner (thickness: 75 $\mu$m) release-treated with silicone. A support was attached to the adhesive layer to provide a tape for transdermal administration. The support was a polyethylene terephthalate (PET) film (thickness: 25 $\mu$m).

The tape for transdermal administration was cut into a piece with an area of 0.7 cm$^2$. The piece was used as a sample for immunity experiment. The release liner was removed before administration.

(Nuclear receptor ligand)

Tretinoin (all-trans-retinoic acid, Wako Pure Chemical Industries, Ltd.)

(Antigen peptide)

HER2/neu_E75 (HER2/neu_E75 peptide, cancer antigen peptide)
IPEP87 (IPEP87 peptide, infectious pathogen-derived antigen)
MAGE-A3_A02 (MAGE3_A02 peptide, cancer antigen peptide)

(Helper peptide)

PADRE

(Adhesive base)

Acrylic base (acrylic adhesive solution prepared by solution polymerization of 75 parts of 2-ethylhexyl acrylate, 22 parts of N-vinyl-2-pyrrolidone, 3 parts of acrylic acid, and 0.2 parts of azobisisobutyronitrile in ethyl acetate at 60°C in an innate gas atmosphere)
PIB (PIB adhesive solution prepared by dissolving 24 parts of polyisobutylene (Oppanol B200, BASF), 36 parts by polyisobutylene (Oppanol B12, BASF), 40 parts of alicyclic petroleum resin (Alkon P-100, Arakawa Chemical Industries, Ltd.) in toluene)

<Evaluation 2>

Each of the tapes for transdermal administration obtained in the examples and the comparative examples were subjected to the following evaluation.

(Evaluation of cellular immunity inducing effect)

The level of induction of antigen-specific cellular immunity was evaluated in the same manner as for the cream for transdermal administration. The results are shown in Fig. 2.

[Table 2]

| No. | Administration route | Dosage form | Base | Antigen | | Nuclear receptor ligand | | Helper peptide | | Immunological evaluation mouse | Immunity result | |
|---|---|---|---|---|---|---|---|---|---|---|---|---|
| | | | | Name | Amount [% by weight] | Name | Amount [% by weight] | Name | Amount [% by weight] | | Average | [cells/well] |
| Comp. Ex. 3 | Transdermal | Tape | Acrylic | HER2/neu_E75 | 10 | - | - | PADRE | 1 | Genetically altered | 7.5 | |
| Ex. 9 | Transdermal | Tape | Acrylic | HER2/neu_E75 | 10 | Tretinoin | 1 | - | 1 | Genetically altered | 72.3 | $2 \times 10^6$ |
| Ex. 10 | Transdermal | Tape | Acrylic | HER2/neu_E75 | 10 | Tretinoin | 1 | PADRE | 1 | Genetically altered | 130.0 | |
| Comp. Ex. 4 | Transdermal | Tape | PIB | HER2/neu_E75 | 10 | - | - | PADRE | 1 | Genetically altered | 7.5 | |
| Ex. 11 | Transdermal | Tape | PIB | HER2/neu_E75 | 10 | Tretinoin | 1 | - | 1 | Genetically altered | 74.5 | $2 \times 10^6$ |
| Ex. 12 | Transdermal | Tape | PIB | HER2/neu_E75 | 10 | Tretinoin | 1 | PADRE | 1 | Genetically altered | 127.5 | |
| Comp- Ex. 5 | Transdermal | Tape | Acrylic | IPEP87 | 10 | - | - | PADRE | 1 | Genetically altered | 5.3 | |
| Ex. 13 | Transdermaf | Tape | Acrylic | IPEP87 | 10 | Tretinoin | 1 | - | 1 | Genetically altered | 76.0 | $2 \times 10^6$ |
| Ex. 14 | Transdermal | Tape | Acrylic | IPEP87 | 10 | Tretinoin | 1 | PADRE | 1 | Genetically altered | 155.3 | |
| Comp. Ex. 6 | Transdermal | Tape | PIB | IPEP87 | 10 | - | - | PADRE | 1 | Genetically altered | 3.8 | |
| Ex. 15 | Transdermal | Tape | PIB | IPEP87 | 10 | Tretinoin | 1 | - | 1 | Genetically altered | 92.3 | $2 \times 10°$ |
| Ex. 16 | Transdermal | Tape | PIB | IPEP87 | 10 | Tretinoin | 1 | PADRE | 1 | Genetically altered | 173.3 | |

(continued)

| No. | Administration route | Dosage form | Base | Antigen | | Nuclear receptor ligand | | Helper peptide | | Immunological evaluation mouse | Immunity result | |
|---|---|---|---|---|---|---|---|---|---|---|---|---|
| | | | | Name | Amount [% by weight] | Name | Amount [% by weight] | Name | Amount [% by weight] | | Average | [cells/well] |
| Comp. Ex. 7 | Transdermal | Tape | Acrylic | MAGE-A3_A02 | 10 | - | - | PADRE | 1 | Genetically altered | 5.8 | $2 \times 10^6$ |
| Ex.17 | Transdermal | Tape | Acrylic | MAGE-A3_A02 | 10 | Tretinoin | 1 | - | 1 | Genetically altered | 90.5 | |
| Ex. 18 | Transdermal | Tape | Acrylic | MAGE-A3_A02 | 10 | Tretinoin | 1 | PADRE | 1 | Genetically altered | 175.5 | |
| Comp. Ex. 8 | Transdermal | Tape | PIB | MAGE-A3_A02 | 10 | - | - | PADRE | 1 | Genetically altered | 4.3 | $2 \times 10^6$ |
| Ex. 19 | Transdermal | Tape | PIB | MAGE-A3_A02 | 10 | Tretinoin | 1 | - | 1 | Genetically altered | 96.0 | |
| Ex. 20 | Transdermal | Tape | PIB | MAGE-A3_A02 | 10 | Tretinoin | 1 | PADRE | 1 | Genetically altered | 186.3 | |

(Examples 21 to 36, Comparative Examples 9 to 10)

(Preparation of solution for transmucosal administration)

A solution for transmucosal administration (transnasal administration or sublingual administration) was prepared according to the formulation shown in Tables 3 and 4 below. Specifically, an antigen (ovalbumin (OVA)) and an immunity induction promoter that was a nuclear receptor ligand were blended in the amounts shown in Tables 3 and 4. For transnasal administration, saline was added thereto such that the amount of the resulting mixture was 10 μL. For sublingual administration, saline was added such that the amount of the resulting mixture was 30 μL. This was followed by mixing to provide a solution for transmucosal administration (transnasal administration or sublingual administration).

(Nuclear receptor ligand)

Tretinoin (all-trans retinoic acid, Wako Pure Chemical Industries, Ltd.)
Isotretinoin (13-cis-retinoic acid, Sigma-Aldrich)
Alitretinoin (9-cis-retinoic acid, Wako Pure Chemical Industries, Ltd.)
Levothyroxine sodium hydrate (Sigma-Aldrich)
Liothyronine (Sigma-Aldrich)
Clomiphene citrate (Sigma-Aldrich)
Raloxifene hydrochloride (LKT Laboratories)
Tamoxifen citrate (Wako Pure Chemical Industries, Ltd.)

(Examples 37 to 52, Comparative Examples 11 and 12)

(Preparation of solid formulation for sublingual administration)

A solid formulation (freeze dry formulation or film) for sublingual administration was prepared according to the formulation shown in Table 5 below. Specifically, an antigen (ovalbumin (OVA)), an immunity induction promoter that was a nuclear receptor ligand, and hydroxypropylcellulose (HPC-SSL, Nippon Soda Co., Ltd.) as a base were blended. Saline was added thereto, followed by mixing to provide a formulation solution. Thereafter, 25 mg of the formulation solution was dispensed, and the dispensed solution was freeze-dried to provide a freeze dry formulation or dried under reduced pressure to provide a film. The immunity induction promoter that was a nuclear receptor ligand was the same as that used for preparing the solution for transmucosal administration.

<Evaluation 3>

Each of the solutions for transmucosal administration and solid formulations for sublingual administration obtained in the examples and the comparative examples was subjected to the following evaluation.

(Evaluation of humoral immunity inducing effect)

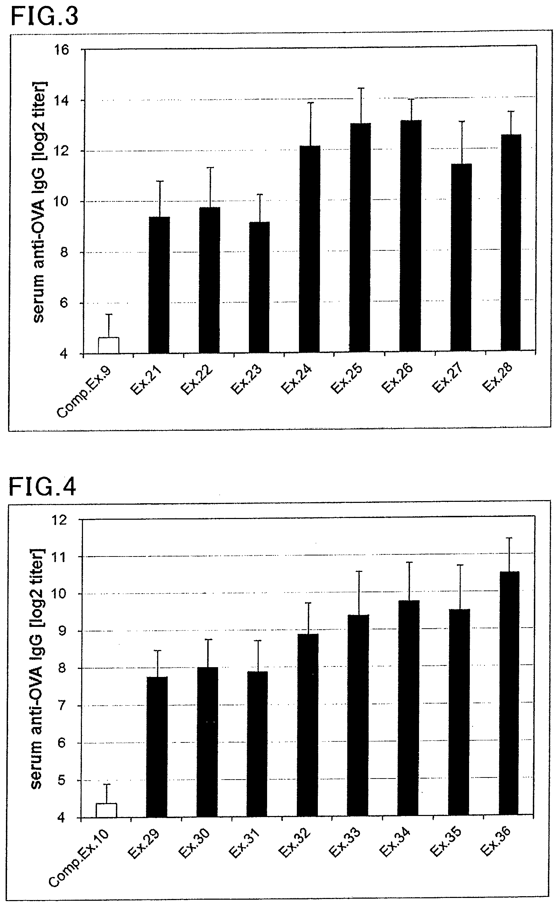

A mouse immunity test using an animal model for immunological evaluation was performed with the solution for transmucosal administration or the solid formulation for sublingual administration by the following procedure. Thereafter, the systemic immune response was evaluated by determining the antigen (OVA)-specific IgG antibody titer in mouse serum. The results are shown in Figs. 3 to 5.

(1) Mouse immunity test of solution for transmucosal administration or solid formulation for sublingual administration

A mouse (BALB/c mouse, female, 7 weeks old) was provided in advance. After the mouse was anesthetized, the solution for transmucosal administration was administered to the mouse by transnasal administration (10 μL, Examples 21 to 28 and Comparative Example 9) or sublingual administration (30 μL, Examples 29 to 36 and Comparative Example 10). Similarly, the solid formulation for sublingual administration (Examples 37 to 52 and Comparative Examples 11 and 12) was administered. One week after the administration, the mouse was anesthetized again, and the administration was performed again in the same manner. One week after the second administration, the mouse serum was taken.

(2) ELISA

(Method for determining antigen-specific IgG antibody titer in mouse serum (ELISA))

To each well of a 96-well plate for ELISA was added 100 μL of an OVA-containing solution (100 μg/mL) diluted with carbonate buffer, followed by standing overnight.

The wells were washed three times with preliminarily prepared wash (Tween 20-containing PBS), and to each well was added 200 μL of a blocking solution prepared by diluting a blocking agent (Block Ace, Sumitomo Dainippon Pharma Co., Ltd.) in purified water to 4 g/100 mL. This was followed by standing for 2 hours at room temperature. The wells were then washed three times with wash.

The serum taken from the mouse was centrifuged at 4°C and 3,000 g for 10 minutes, and the supernatant was recovered. The supernatant was diluted in two-fold increments using a solution prepared by diluting a blocking agent in a phosphate buffer (Nacalai Tesque, Inc.) to 0.4 g/100 mL. The diluted solution was added to wells (50 μL for each well), followed by standing for 2 hours at room temperature.

The wells were then washed three times with wash. An HRP-labeled anti-mouse IgG antibody (Goat-anti mouse IgG Fc HRP, BETHYL) was diluted 10,000-fold using a solution prepared by diluting a blocking agent in a phosphate buffer (Nacalai Tesque, Inc.) to 0.4 g/100 mL. To each well was added 100 μL of the resulting solution, followed by standing for 1 hour at room temperature.

The wells were then washed three times with wash, and 100 μL of a TMB solution (ELISA POD TMB kit, Nacalai Tesque, Inc.) was added to each well, followed by standing for 30 minutes at dark place.

Thereafter, 100 μL of a 1M sulfuric acid solution was added to each well, and the 96-well plate was subjected to measurement of absorbance at 450 nm with a microplate reader (Spectra Max M2$^e$, molecular device). The IgG antibody titer in the mouse serum was determined as Log2 titer based on the absorbance at the incremental dilution.

[Table 3]

| No. | Administration route | Dosage form | Antigen | | Nuclear receptor ligand | | Pharmacological effect | Immunological evaluation mouse | IgG antibody titer [Log2 titer] |
|---|---|---|---|---|---|---|---|---|---|
| | | | Name | Amount [ug] | Name | Amount [ug] | | | |
| Comp. Ex. 9 | Transnasal | Solution | OVA | 1 | - | - | - | BALB/c | 4.6 |
| Ex. 21 | Transnasal | Solution | OVA | 1 | Tretinoin | 50 | RAR activation | BALB/c | 9.4 |
| Ex. 22 | Transnasal | Solution | OVA | 1 | Isotretinoin | 50 | RAR activation | BALB/c | 9.8 |
| Ex. 23 | Transnasal | Solution | OVA | 1 | Alitretinoin | 50 | RAR activation | BALB/c | 9.1 |
| Ex. 24 | Transnasal | Solution | OVA | 1 | Levothyroxine sodium | 20 | TR activation | BALB/c | 12.1 |
| Ex. 25 | Transnasal | Solution | OVA | 1 | Liothyronine | 50 | TR activation | BALB/c | 13.0 |
| Ex. 26 | Transnasal | Solution | OVA | 1 | Clomiphene citrate | 10 | ER modulation | BALB/c | 13.1 |
| Ex. 27 | Transnasal | Solution | OVA | 1 | Raloxifene hydrochloride | 20 | ER modulation | BALB/c | 11.4 |
| Ex. 28 | Transnasal | Solution | OVA | 1 | Tamoxifen citrate | 10 | ER modulation | BALB/c | 12.5 |

[Table 4]

| No. | Administration route | Dosage form | Antigen | | Nuclear receptor ligand | | Pharmacological effect | Immunological evaluation mouse | IgG antibody titer [Log2 titer] |
|---|---|---|---|---|---|---|---|---|---|
| | | | Name | Amount [ug] | Name | Amount [ug] | | | |
| Comp. Ex. 10 | Sublingual | Solution | OVA | 1 | - | - | - | BALB/c | 4.4 |
| Ex. 29 | Sublingual | Solution | OVA | 1 | Tretinoin | 100 | RAR activation | BALB/c | 7.8 |
| Ex. 30 | Sublingual | Solution | OVA | 1 | Isotretinoin | 100 | RAR activation | BALB/c | 8.0 |
| Ex. 31 | Sublingual | Solution | OVA | 1 | Alitretinoin | 100 | RAR activation | BALB/c | 7.9 |
| Ex. 32 | Sublingual | Solution | OVA | 1 | Levothyroxine sodium | 100 | TR activation | BALB/c | 8.9 |
| Ex. 33 | Sublingual | Solution | OVA | 1 | Liothyronine | 100 | TR activation | BALB/c | 9.4 |
| Ex. 34 | Sublingual | Solution | OVA | 1 | Clomiphene citrate | 100 | ER modulation | BALB/c | 9.8 |
| Ex. 35 | Sublingual | Solution | OVA | 1 | Raloxifene hydrochloride | 100 | ER modulation | BALB/c | 9.5 |
| Ex. 36 | Sublingual | Solution | OVA | 1 | Tamoxifen citrate | 100 | ER modulation | BALB/c | 10.5 |

[Table 5]

| Component | | | Formulation [Parts by weight] | | | | | | | | | | | | | | | | | |
|---|---|---|---|---|---|---|---|---|---|---|---|---|---|---|---|---|---|---|---|---|
| | | | Ex. | | | | | | | | | | | | | | | | Comp. Ex. | |
| | | | 37 | 38 | 39 | 40 | 41 | 42 | 43 | 44 | 45 | 46 | 47 | 48 | 49 | 50 | 51 | 52 | 11 | 12 |
| Antigen | | OVA | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Immunostimulant | Nuclear receptor ligand | Tretinoin | 10 | - | - | - | - | - | - | - | 10 | - | - | - | - | - | - | - | - | - |
| | | Isotretinoin | - | 10 | - | - | - | - | - | - | - | 10 | - | - | - | - | - | - | - | - |
| | | Alitretinoin | - | - | 10 | - | - | - | - | - | - | - | 10 | - | - | - | - | - | - | - |
| | | Levothyroxine sodium | - | - | - | 10 | - | - | - | - | - | - | - | 10 | - | - | - | - | - | - |
| | | Liothyronine | - | - | - | - | 10 | - | - | - | - | - | - | - | 10 | - | - | - | - | - |
| | | Clomiphene citrate | - | - | - | - | - | 10 | - | - | - | - | - | - | - | 10 | - | - | - | - |
| | | Raloxifene hydrochlo-ride | - | - | - | - | - | - | 10 | - | - | - | - | - | - | - | 10 | - | - | - |
| | | Tamoxifen ci-trate | - | - | - | - | - | - | - | 10 | - | - | - | - | - | - | - | 10 | - | - |
| Base | | HPC-SSL | 250 | 250 | 250 | 250 | 250 | 250 | 250 | 250 | 250 | 250 | 250 | 250 | 250 | 250 | 250 | 250 | 250 | 250 |
| Saline solution | | | 739.9 | 739.9 | 739.9 | 739.9 | 739.9 | 739.9 | 739.9 | 739.9 | 739.9 | 739.9 | 739.9 | 739.9 | 739.9 | 739.9 | 739.9 | 739.9 | 749.9 | 749.9 |
| Dispensing amount [mg/mouse] | | | 25 | 25 | 25 | 25 | 25 | 25 | 25 | 25 | 25 | 25 | 25 | 25 | 25 | 25 | 25 | 25 | 25 | 25 |
| IgGantibody titer [Log2 titer] | | | 7.9 | 8.0 | 8.1 | 8.9 | 9.6 | 10.0 | 9.5 | 10.8 | 7.8 | 7.9 | 8.1 | 9.0 | 9.6 | 10.0 | 9.6 | 10.8 | 4.3 | 4.3 |
| Dosage form | | | Solid (freeze dry) | | | | | | | | Film | | | | | | | | Solid (freeze dry) | Film |
| Administration route | | | Sublingual administration | | | | | | | | | | | | | | | | | |

(Examples 53 to 56, Comparative Example 13)

(Preparation of solution for subcutaneous administration)

A formulation for subcutaneous administration was prepared according to the formulation shown in Table 6 below.

Specifically, an antigen (ovalbumin (OVA)) and an immunity induction promoter that was a nuclear receptor ligand were blended in the amounts shown in Table 6. Saline was added thereto such that the amount of the resulting mixture was 200 μL, followed by mixing to provide a solution for subcutaneous administration.

<Evaluation 4>

Each of the formulations for subcutaneous administration obtained in the examples and the comparative examples was subjected to the following evaluation.

(Evaluation of humoral immunity inducing effect)

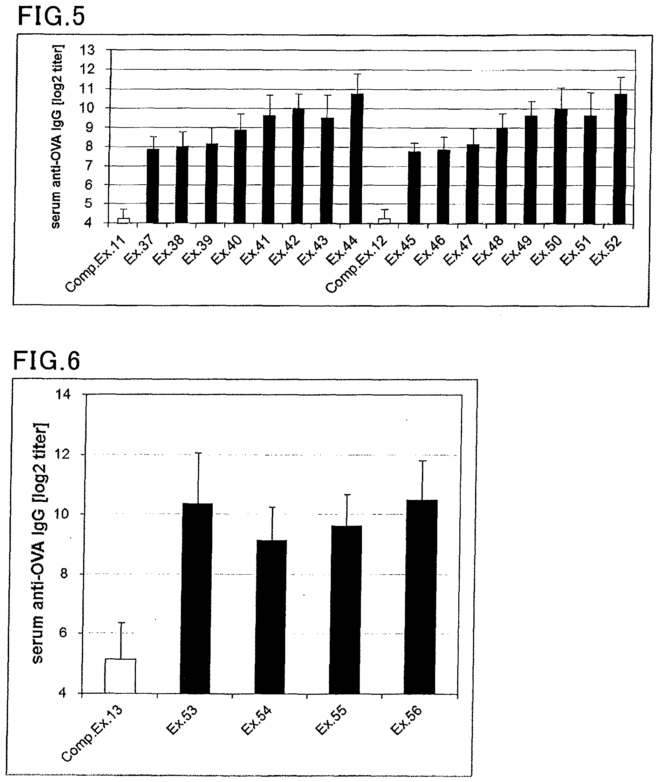

A mouse immunity test using an animal model for immunological evaluation was performed with the formulation for subcutaneous administration by the following procedure. Thereafter, the systemic immune response was evaluated by determining the antigen (OVA)-specific IgG antibody in mouse serum. The results are shown in Fig. 6.

(1) Mouse immunity test of formulation for subcutaneous administration

A mouse (BALB/c mouse, female, 7 weeks old) was provided in advance. After the mouse was anesthetized, 200 μL of the formulation was subcutaneously administered to the skin of the back of the mouse. One week after the administration, the mouse was anesthetized again, and administration was performed again in the same manner. One week after the second administration, the mouse serum was taken.

(2) ELISA

The antigen (OVA)-specific IgG antibody titer in the mouse serum was determined by ELISA by the same procedure as in <Evaluation 3>.

[Table 6]

| No. | Administration route | Dosage form | Antigen | | Nuclear receptor ligand | | Pharmacological effect | Immunological evaluation mouse | IgG antibody titer [Log2 titer] |
|---|---|---|---|---|---|---|---|---|---|
| | | | Name | Amount [ug] | Name | Amount [ug] | | | |
| Comp. Ex. 13 | Subcutaneous | Solution | OVA | 0.05 | - | - | - | BALB/c | 5.1 |
| Ex. 53 | Subcutaneous | Solution | OVA | 0.05 | Tretinoin | 200 | RAR activation | BALB/c | 10.4 |
| Ex. 54 | Subcutaneous | Solution | OVA | 0.05 | Levothyroxine sodium | 200 | TR activation | BALB/c | 9.1 |
| Ex. 55 | Subcutaneous | Solution | OVA | 0.05 | Liothyronine | 200 | TR activation | BALB/c | 9.6 |
| Ex. 56 | Subcutaneous | Solution | OVA | 0.05 | Raloxifene hydrochloride | 200 | ER modulation | BALB/c | 10.5 |

(Examples 57 to 60, Comparative Example 14)

(Preparation of cream for transdermal administration)

A cream for transdermal administration was prepared according to the formulation shown in Table 7 below. Specifically, an antigen (ovalbumin (OVA)) and a nuclear receptor ligand were blended in the amounts shown in Table 7 and a base (base cream) was added thereto to achieve a total weight of 100 parts by weight. This was followed by mixing to provide a cream for transdermal administration. The base cream was prepared by blending and mixing materials according to the formulation shown in Table 14.

The immunity induction promoter that was a nuclear receptor ligand was the same as that used for preparing the solutions for transnasal or sublingual administration. White Vaseline, sorbitan monostearate, isostearic acid, benzyl alcohol, stearyl alcohol, polysorbate 60, and concentrated glycerin were purchased from Wako Pure Chemical Industries, Ltd. Cetanol was purchased from Tokyo Chemical Industry Co., Ltd.

A composite base was prepared by bonding a PET film/PET nonwoven fabric laminate (area: 0.7 cm$^2$) to the center portion of an adhesive tape for attachment such that the PET-film side faced the tape. The cream in an amount of 4 mg was applied to the nonwoven fabric portion of the composite base to provide a sample for a mouse immunity test.

<Evaluation 5>

Each of the creams for transdermal administration obtained in the examples and the comparative examples was subjected to the following evaluation.

(Evaluation of humoral immunity inducing effect)

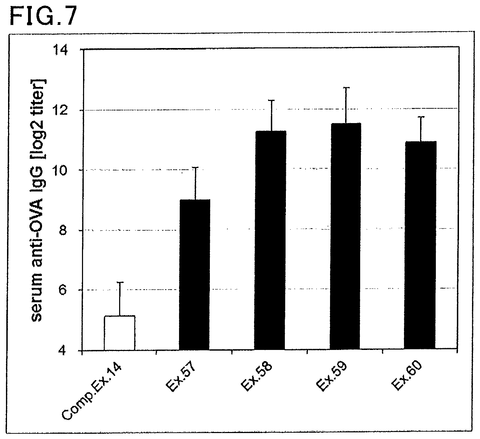

A mouse immunity test using an animal model for immunological evaluation was performed with the cream for transdermal administration by the following procedure. Thereafter, the systemic immune response was evaluated by determining the antigen (OVA)-specific IgG antibody in mouse serum. The results are shown in Fig. 7.

(1) Mouse immunity test of cream for transdermal administration

The right back of a mouse (C57BL6 NCr mouse, female, 7 weeks old) was shaved in advance. After a rearing period for recovery from the skin damage caused by the shaving, 4 mg of the cream for transdermal administration was administered to the skin of the right back of the mouse, and the left back was shaved at the same time. Twenty-four hours later, the cream for transdermal administration on the right back was removed. One week after the administration, the cream for transdermal administration was administered to the skin of the left back of the mouse in the same manner and removed 24 hours later. One week after the second administration, the mouse serum was taken.

(2) ELISA

The antigen (OVA)-specific IgG antibody titer in the mouse serum was determined by ELISA by the same procedure as in <Evaluation 3>.

[Table 7]

| No. | Administration route | Dosage form | Antigen | | Nuclear receptor ligand | | Pharmacological effect | Immunological evaluation mouse | IgG antibody titer [Log2 titer] |
|---|---|---|---|---|---|---|---|---|---|
| | | | Name | Amount [Parts by weight] | Name | Amount [Parts by weight] | | | |
| Comp. Ex. 14 | Transdermal | Cream | OVA | 5 | - | - | - | C57BL6 | 5.1 |
| Ex. 57 | Transdermal | Cream | OVA | 5 | Tretinoin | 5 | RAR activation | C57BL6 | 9.0 |
| Ex. 58 | Transdermal | Cream | OVA | 5 | Levothyroxine sodium | 5 | TR activation | C57BL6 | 11.3 |
| Ex. 59 | Transdermal | Cream | OVA | 5 | Liothyronine | 5 | TR activation | C57BL6 | 11.5 |
| Ex. 60 | Transdermal | Cream | OVA | 5 | Raloxifene hydrochloride | 5 | ER modulation | C57BL6 | 10.9 |

(Examples 61 to 180, Comparative Examples 15 to 54)

A solution for transmucosal administration (transnasal administration or sublingual administration) was prepared according to the formulation shown in Tables 8 to 12 below. Specifically, an antigen and a nuclear receptor ligand were blended in the amounts shown in Tables 8 to 12. For transnasal administration, saline was added thereto so that the amount of the resulting mixture was 10 $\mu$L. For sublingual administration, saline was added so that the amount of the resulting mixture was 30 $\mu$L. This was followed by mixing to provide a solution for transmucosal administration (transnasal administration or sublingual administration).

Influenza vaccine antigens used were an influenza vaccine antigen-containing solution H1N1 (A/California/07/2009, The Research Foundation for Microbial Diseases of Osaka University), H3N2 (A/Victoria361/2011, The Research Foundation for Microbial Diseases of Osaka University), Influenza B virus (B/Wisconsin/1/2010, The Research Foundation for Microbial Diseases of Osaka University), Influenza B virus (B/Brisbane/60/2008, The Research Foundation for Microbial Diseases of Osaka University) were used. Also used were a pneumococcal capsular polysaccharide-containing solution (Pneumovax NP, MSD), HPV16 recombinant protein-containing solution (HPV16, PROSPEC), a live attenuated rotavirus-containing solution (RotaTeq Oral Solution, MSD), an inactivated poliovirus-containing solution (IMOVAX POLIO for subcutaneous injection, Sanofi), an inactivated hepatitis A virus-containing solution (Aimmugen, The Chemo-Sero-Therapeutic Research Institute), an inactivated Japanese encephalitis virus-containing solution (Encevac for subcutaneous injection, The Chemo-Sero-. Therapeutic Research Institute), a live attenuated mumps virus-containing solution (live mumps vaccine, Kitasato Daiichi Sankyo Vaccine Co., Ltd), a live attenuated measles virus-containing solution (live measles vaccine, Kitasato Daiichi Sankyo Vaccine Co., Ltd), a live attenuated rubella virus-containing solution (dried live attenuated rubella vaccine, Kitasato Daiichi Sankyo Vaccine Co., Ltd), a solution containing tetanus toxoid-conjugated haemophilus influenzae type b polysaccharide (ActHIB, Sanofi), a recombinant HBs antigen protein-containing solution (Bimmugen, The Chemo-Sero-Therapeutic Research Institute), a live attenuated yellow fever virus-containing solution (yellow fever vaccine, Sanofi), a tetanus toxoid-containing solution (tetanus toxoid, Denka Seiken Co., Ltd.), a live attenuated varicella virus-containing solution (dried live attenuated varicella vaccine, The Research Foundation for Microbial Diseases of Osaka University), a live BCG-containing solution (dried BCG vaccine, Japan BCG Laboratory), and an inactivated rabies virus-containing solution (tissue-cultured inactivated rabies vaccine, The Chemo-Sero-Therapeutic Research Institute).

As the immunity induction promoter that is a nuclear receptor ligand, tretinoin (all-trans-retinoic acid, Wako Pure Chemical Industries, Ltd.), liothyronine (Sigma-Aldrich), and tamoxifen citrate (Wako Pure Chemical Industries, Ltd.) were used.

<Evaluation 6>

Each of the solutions for transmucosal administration obtained in the examples and the comparative examples was subjected to the following evaluation.

(Evaluation of humoral immunity inducing effect)

A mouse immunity test using an animal model for immunological evaluation was performed with the solution for transmucosal administration by the following procedure. Thereafter, the systemic immune response was evaluated by determining the antigen (OVA)-specific IgG antibody in mouse serum.

(1) Mouse immunity test of solution for transmucosal administration

Mouse serum was taken by the same procedure as in <Evaluation 3>, an evaluation of solution for transmucosal administration or sublingual administration.

(2) ELISA

The antigen (OVA)-specific IgG antibody titer in the mouse serum was determined by ELISA by the same procedure as in <Evaluation 3>, an evaluation of solution for transmucosal administration or sublingual administration.

The evaluation of the humoral immunity inducing effect shows that the transmucosal administration (transnasal administration or sublingual administration) of a solution for transmucosal administration containing an immunity induction promoter that is a nuclear receptor ligand (Examples 21 to 36) provides a higher antigen-specific IgG antibody titer than the administration of a solution for transmucosal administration free from an immunity induction promoter that is a nuclear receptor ligand (Comparative Examples 9 and 10).

Accordingly, also when antigens such as those shown in Tables 8 to 12 below are used, the use of an immunity induction promoter that is a nuclear receptor ligand leads to a high antigen-specific IgG antibody titer.

[Table 8]

| | Antigen | | Nuclear receptor ligand | | Formulation | Administration route | Amount [μL] |
|---|---|---|---|---|---|---|---|
| | Name | Amount [μg] | Name | Amount [μg] | | | |
| Comp. Ex. 15 | A/California/07/2009 [H1N1] | 1.0 | - | - | Solution | Transnasal | 10 |
| Ex. 61 | A/California/07/2009 [H1N1] | 1.0 | Tretinoin | 50 | Solution | Transnasal | 10 |
| Ex. 62 | A/California/07/2009 [H1N1] | 1.0 | Liothyronine | 50 | Solution | Transnasal | 10 |
| Ex. 63 | A/California/07/2009 [H1N1] | 1.0 | Tamoxifen | 10 | Solution | Transnasal | 10 |
| Comp. Ex. 16 | A/California/07/2009 [H1N1] | 1.0 | - | - | Solution | Sublingual | 30 |
| Ex. 64 | A/California/07/2009 [H1N1] | 1.0 | Tretinoin | 100 | Solution | Sublingual | 30 |
| Ex. 65 | A/California/07/2009 [H1N1] | 1.0 | Liothyronine | 100 | Solution | Sublingual | 30 |
| Ex. 66 | A/California/07/2009 [H1N1] | 1.0 | Tamoxifen | 100 | Solution | Sublingual | 30 |
| Comp. Ex. 17 | A/Victoria361/2011 [H3N2] | 1.0 | - | - | Solution | Transnasal | 10 |
| Ex. 67 | A/Victoria361/2011 [H3N2] | 1.0 | Tretinoin | 50 | Solution | Transnasal | 10 |
| Ex. 68 | A/Victoria361/2011 [H3N2] | 1.0 | Liothyronine | 50 | Solution | Transnasal | 10 |
| Ex. 69 | A/Victoria361/2011 [H3N2] | 1.0 | Tamoxifen | 10 | Solution | Transnasal | 10 |
| Comp. Ex. 18 | A/Victoria361/2011 [H3N2] | 1.0 | - | - | Solution | Sublingual | 30 |
| Ex.70 | A/Victoria361/2011 [H3N2] | 1.0 | Tretinoin | 100 | Solution | Sublingual | 30 |
| Ex. 71 | A/Victoria361/2011 [H3N2] | 1.0 | Liothyronine | 100 | Solution | Sublingual | 30 |
| Ex. 72 | A/Victoria361/2011 [H3N2] | 1.0 | Tamoxifen | 100 | Solution | Sublingual | 30 |
| Comp. Ex. 19 | B/Wisconsin/1/2010 | 1.0 | - | - | Solution | Transnasal | 10 |
| Ex. 73 | B/Wisconsin/1/2010 | 1.0 | Tretinoin | 50 | Solution | Transnasal | 10 |
| Ex. 74 | B/Wisconsin/1/2010 | 1.0 | Liothyronine | 50 | Solution | Transnasal | 10 |
| Ex. 75 | B/Wisconsin/1/2010 | 1.0 | Tamoxifen | 10 | Solution | Transnasal | 10 |
| Comp. Ex. 20 | B/Wisconsin/1/2010 | 1.0 | - | - | Solution | Sublingual | 30 |
| Ex.76 | B/Wisconsin/1/2010 | 1.0 | Tretinoin | 100 | Solution | Sublingual | 30 |
| Ex. 77 | B/Wisconsin/1/2010 | 1.0 | Liothyronine | 100 | Solution | Sublingual | 30 |
| Ex. 78 | B/Wisconsin/1/2010 | 1.0 | Tamoxifen | 100 | Solution | Sublingual | 30 |

(continued)

| | Antigen | | Nuclear receptor ligand | | Formulation | Administration route | Amount [$\mu$L] |
|---|---|---|---|---|---|---|---|
| | Name | Amount [$\mu$g] | Name | Amount [$\mu$g] | | | |
| Comp. Ex. 21 | B/Brisbane/60/2008 | 1.0 | - | - | Solution | Transnasal | 10 |
| Ex. 79 | B/Brisbane/60/2008 | 1.0 | Tretinoin | 50 | Solution | Transnasal | 10 |
| Ex. 80 | B/Brisbane/60/2008 | 1.0 | Liothyronine | 50 | Solution | Transnasal | 10 |
| Ex. 81 | B/Brisbane/60/2008 | 1.0 | Tamoxifen | 10 | Solution | Transnasal | 10 |
| Comp. Ex. 22 | B/Brisbane/60/2008 | 1.0 | - | - | Solution | Sublingual | 30 |
| Ex. 82 | B/Brisbane/60/2008 | 1.0 | Tretinoin | 100 | Solution | Sublingual | 30 |
| Ex. 83 | B/Brisbane/60/2008 | 1.0 | Liothyronine | 100 | Solution | Sublingual | 30 |
| Ex. 84 | B/Brisbane/60/2008 | 1.0 | Tamoxifen | 100 | Solution | Sublingual | 30 |

[Table 9]

| | Antigen | | Nuclear receptor ligand | | Formulation | Administration route | Amount [μL] |
|---|---|---|---|---|---|---|---|
| | Name | Amount [μg] | Name | Amount [μg] | | | |
| Comp. Ex. 23 | Pneumococcal capsular polysaccharide Pneumovax NP | 20 | - | - | Solution | Transnasal | 10 |
| Ex. 85 | Pneumococcal capsular polysaccharide Pneumovax NP | 20 | Tretinoin | 50 | Solution | Transnasal | 10 |
| Ex. 86 | Pneumococcal capsular polysaccharide Pneumovax NP | 20 | Liothyronine | 50 | Solution | Transnasal | 10 |
| Ex. 87 | Pneumococcal capsular polysaccharide Pneumovax NP | 20 | Tamoxifen | 10 | Solution | Transnasal | 10 |
| Comp. Ex. 24 | Pneumococcal capsular polysaccharide Pneumovax NP | 20 | - | - | Solution | Sublingual | 30 |
| Ex. 88 | Pneumococcal capsular polysaccharide Pneumovax NP | 20 | Tretinoin | 100 | Solution | Sublingual | 30 |
| Ex. 89 | Pneumococcal capsular polysaccharide Pnaumovax NP | 20 | Liothyronine | 100 | Solution | Sublingual | 30 |
| Ex. 90 | Pneumococcal capsular polysaccharide Pneumovax NP | 20 | Tamoxifen | 100 | Solution | Sublingual | 30 |
| Comp. Ex. 25 | HPV16 recombinant protein | 10 | - | - | Solution | Transnasal | 10 |
| Ex. 91 | HPV16 recombinant protein | 10 | Tretinoin | 50 | Solution | Transnasal | 10 |
| Ex. 92 | HPV16 recombinant protein | 10 | Liothyronine | 50 | Solution | Transnasal | 10 |
| Ex. 93 | HPV16 recombinant protein | 10 | Tamoxifen | 10 | Solution | Transnasal | 10 |

(continued)

| | Antigen | | Nuclear receptor ligand | | Formulation | Administration route | Amount [$\mu$L] |
|---|---|---|---|---|---|---|---|
| | Name | Amount [$\mu$g] | Name | Amount [$\mu$g] | | | |
| Comp. Ex. 26 | HPV16 recombinant protein | 10 | - | - | Solution | Sublingual | 30 |
| Ex. 94 | HPV16 recombinant protein | 10 | Tretinoin | 100 | Solution | Sublingual | 30 |
| Ex. 95 | HPV16 recombinant protein | 10 | Liothyronine | 100 | Solution | Sublingual | 30 |
| Ex. 96 | HPV16 recombinant protein | 10 | Tamoxifen | 100 | Solution | Sublingual | 30 |
| Comp. Ex. 27 | Live attenuated rotavirus (RIX4414 strain) | 10 | - | - | Solution | Transnasal | 10 |
| Ex. 97 | Live attenuated rotavirus (RIX4414 strain) | 10 | Tretinoin | 50 | Solution | Transnasal | 10 |
| Ex. 98 | Live attenuated rotavirus (RIX4414 strain) | 10 | Liothyronine | 50 | Solution | Transnasal | 10 |
| Ex. 99 | Live attenuated rotavirus (RIX4414 strain) | 10 | Tamoxifen | 10 | Solution | Transnasal | 10 |
| Comp. Ex. 28 | Live attenuated rotavirus (RIX4414 strain) | 10 | - | - | Solution | Sublingual | 30 |
| Ex. 100 | Live attenuated rotavirus (RIX4414 strain) | 10 | Tretinoin | 100 | Solution | Sublingual | 30 |
| Ex. 101 | Live attenuated rotavirus (RIX4414 strain) | 10 | Liothyronine | 100 | Solution | Sublingual | 30 |
| Ex. 102 | Live attenuated rotavirus (RIX4414 strain) | 10 | Tamoxifen | 100 | Solution | Sublingual | 30 |
| Comp. Ex. 29 | Inactivated poliovirus(type 1, type 2, and type 3) | Vaccine 100 $\mu$L equivalent | - | - | Solution | Transnasal | 10 |
| Ex. 103 | Inactivated poliovirus(type 1, type 2, and type 3) | Vaccine 100 $\mu$L equivalent | Tretinoin | 50 | Solution | Transnasal | 10 |

(continued)

| | | Antigen | | Nuclear receptor ligand | | Formulation | Administration route | Amount [μL] |
|---|---|---|---|---|---|---|---|---|
| | | Name | Amount [μg] | Name | Amount [μg] | | | |
| Ex. 104 | | Inactivated poliovirus (type 1, type 2, and type 3) | Vaccine 100 μL equivalent | Liothyronine | 50 | Solution | Transnasal | 10 |
| Ex. 105 | | Inactivated poliovirus (type 1, type 2. and type 3) | Vaccine 100 μL equivalent | Tamoxifen | 10 | Solution | Transnasal | 10 |
| Comp. Ex. 30 | | Inactivated poliovirus (type 1, type 2, and type 3) | Vaccine 100 μL equivalent | - | - | Solution | Sublingual | 30 |
| Ex. 106 | | Inactivated poliovirus (type 1, type 2, and type 3) | Vaccine 100 μL equivalent | Tretinoin | 100 | Solution | Sublingual | 30 |
| Ex. 107 | | Inactivated poliovirus (type 1, type 2, and type 3) | Vaccine 100 μL equivalent | Liothyronine | 100 | Solution | Sublingual | 30 |
| Ex. 108 | | Inactivated poliovirus (type 1, type 2, and type 3) | Vaccine 100 μL equivalent | Tamoxifen | 100 | Solution | Sublingual | 30 |

[Table 10]

| | Antigen | | Nuclear receptor ligand | | Formulation | Administration route | Amount [μL] |
|---|---|---|---|---|---|---|---|
| | Name | Amount [μg] | Name | Amount [μg] | | | |
| Comp. Ex. 31 | Inactivated hepatitis A virus | Vaccine 100 μL equivalent | - | - | Solution | Transnasal | 10 |
| Ex. 109 | Inactivated hepatitis A virus | Vaccine 100 μL equivalent | Tretinoin | 50 | Solution | Transnasal | 10 |
| Ex. 110 | Inactivated hepatitis A virus | Vaccine 100 μL equivalent | Liothyronine | 50 | Solution | Transnasal | 10 |
| Ex. 111 | Inactivated hepatitis A virus | Vaccine 100 μL equivalent | Tamoxifen | 10 | Solution | Transnasal | 10 |
| Comp. Ex. 32 | Inactivated hepatitis A virus | Vaccine 100 μL equivalent | - | - | Solution | Sublingual | 30 |

(continued)

| | | Antigen | | Nuclear receptor ligand | | Formulation | Administration route | Amount [μL] |
|---|---|---|---|---|---|---|---|---|
| | | Name | Amount [μg] | Name | Amount [μg] | | | |
| Ex. 112 | | Inactivated hepatitis A virus | Vaccine 100 μL equivalent | Tretinoin | 100 | Solution | Sublingual | 30 |
| Ex. 113 | | Inactivated hepatitis A virus | Vaccine 100 μL equivalent | Liothyronine | 100 | Solution | Sublingual | 30 |
| Ex. 114 | | Inactivated hepatitis A virus | Vaccine 100 μL equivalent | Tamoxifen | 100 | Solution | Sublingual | 30 |
| Comp. Ex. 33 | | Inactivated Japanese encephalitis virus | Vaccine 100 μL equivalent | - | - | Solution | Transnasal | 10 |
| Ex. 115 | | Inactivated Japanese encephalitis virus | Vaccine 100 μL equivalent | Tretinoin | 50 | Solution | Transnasal | 10 |
| Ex. 116 | | Inactivated Japanese encephalitis virus | Vaccine 100 μL equivalent | Liothyronine | 50 | Solution | Transnasal | 10 |
| Ex. 117 | | Inactivated Japanese encephalitis virus | Vaccine 100 μL equivalent | Tamoxifen | 10 | Solution | Transnasal | 10 |
| Comp. Ex. 34 | | Inactivated Japanese encephalitis virus | Vaccine 100 μL equivalent | - | - | Solution | Sublingual | 30 |
| Ex. 118 | | Inactivated Japanese encephalitis virus | Vaccine 100 μL equivalent | Tretinoin | 100 | Solution | Sublingual | 30 |
| Ex. 119 | | Inactivated Japanese encephalitis virus | Vaccine 100 μL equivalent | Liothyronine | 100 | Solution | Sublingual | 30 |
| Ex. 120 | | Inactivated Japanese encephalitis virus | Vaccine 100 μL equivalent | Tamoxifen | 100 | Solution | Sublingual | 30 |
| Comp. Ex. 35 | | Live attenuated mumps virus | Vaccine 100 μL equivalent | - | - | Solution | Transnasal | 10 |

(continued)

| | Antigen | | Nuclear receptor ligand | | Formulation | Administration route | Amount [$\mu$L] |
|---|---|---|---|---|---|---|---|
| | Name | Amount [$\mu$g] | Name | Amount [$\mu$g] | | | |
| Ex. 121 | Live attenuated mumps virus | Vaccine 100 $\mu$L equivalent | Tretinoin | 50 | Solution | Transnasal | 10 |
| Ex. 122 | Live attenuated mumps virus | Vaccine 100 $\mu$L equivalent | Liothvronine | 50 | Solution | Transnasal | 10 |
| Ex. 123 | Live attenuated mumps virus | Vaccine 100 $\mu$L equivalent | Tamoxifen | 10 | Solution | Transnasal | 10 |
| Como. Ex. 36 | Live attenuated mumos virus | Vaccine 100 $\mu$L equivalent | - | - | Solution | Sublingual | 30 |
| Ex. 124 | Live attenuated mumps virus | Vaccine 100 $\mu$L equivalent | Tretinoin | 100 | Solution | Sublingual | 30 |
| Ex. 125 | Live attenuated mumps virus | Vaccine 100 $\mu$L equivalent | Liothyronine | 100 | Solution | Sublingual | 30 |
| Ex. 126 | Live attenuated mumps virus | Vaccine 100 $\mu$L equivalent | Tamoxifen | 100 | Solution | Sublingual | 30 |
| Comp. Ex. 37 | Live attenuated measles virus | Vaccine 100 $\mu$L equivalent | - | - | Solution | Transnasal | 10 |
| Ex. 127 | Live attenuated measles virus | Vaccine 100 $\mu$L equivalent | Tretinoin | 50 | Solution | Transnasal | 10 |
| Ex. 128 | Live attenuated measles virus | Vaccine 100 $\mu$L equivalent | Liothyronine | 50 | Solution | Transnasal | 10 |
| Ex. 129 | Live attenuated measles virus | Vaccine 100 $\mu$L equivalent | Tamoxifen | 10 | Solution | Transnasal | 10 |
| Comp. Ex. 38 | Live attenuated measles virus | Vaccine 100 $\mu$L equivalent | - | - | Solution | Sublingual | 30 |
| Ex. 130 | Live attenuated measles virus | Vaccine 100 $\mu$L equivalent | Tretinoin | 100 | Solution | Sublingual | 30 |
| Ex. 131 | Live attenuated measles virus | Vaccine 100 $\mu$L equivatent | Liothyronine | 100 | Solution | Sublingual | 30 |
| Ex. 132 | Live attenuated measles virus | Vaccine 100 $\mu$L equivalent | Tamoxifen | 100 | Solution | Sublingual | 30 |

[Table 11]

| | Antigen | | Nuclear receptor ligand | | Formulation | Administration route | Amount [$\mu$L] |
| | Name | Amount [$\mu$g] | Name | Amount [$\mu$g] | | | |
|---|---|---|---|---|---|---|---|
| Comp. Ex. 39 | Live attenuated rubella virus | Vaccine 100 $\mu$L equivalent | - | - | Solution | Transnasal | 10 |
| Ex. 133 | Live attenuated rubella virus | Vaccine 100 $\mu$L equivalent | Tretinoin | 50 | Solution | Transnasal | 10 |
| Ex. 134 | Live attenuated rubella virus | Vaccine 100 $\mu$L equivalent | Liothyronine | 50 | Solution | Transnasal | 10 |
| Ex. 135 | Live attenuated rubella virus | Vaccine 100 $\mu$L equivalent | Tamoxifen | 10 | Solution | Transnasal | 10 |
| Comp. Ex. 40 | Live attenuated rubella virus | Vaccine 100 $\mu$L equivalent | - | - | Solution | Sublingual | 30 |
| Ex. 136 | Live attenuated rubella virus | Vaccine 100 $\mu$L equivalent | Tretinoin | 100 | Solution | Sublingual | 30 |
| Ex. 137 | Live attenuated rubella virus | Vaccine 100 $\mu$L equivalent | Liothyronine | 100 | Solution | Sublingual | 30 |
| Ex. 138 | Live attenuated rubella virus | Vaccine 100 $\mu$L equivalent | Tamoxifen | 100 | Solution | Sublingual | 30 |
| Comp. Ex. 41 | Tetanus toxoid-conjugated Haemophilus influenzae type b polysaccharide | Vaccine 100 $\mu$L equivalent | - | - | Solution | Transnasal | 10 |
| Ex. 139 | Tetanus toxoid-conjugated Haemophilus influenzae type b polysaccharide | Vaccine 100 $\mu$L equivalent | Tretinoin | 50 | Solution | Transnasal | 10 |
| Ex. 140 | Tetanus toxoid-conjugated Haemophilus influenzae type b polysaccharide | Vaccine 100 $\mu$L equivalent | Liothyronine | 50 | Solution | Transnasal | 10 |
| Ex. 141 | Tetanus toxoid-conjugated Haemophilus influenzae type b polysaccharide | Vaccine 100 $\mu$L equivalent | Tamoxifen | 10 | Solution | Transnasal | 10 |
| Comp. Ex. 42 | Tetanus toxoid-conjugated Haemophilus influenzae type b polysaccharide | Vaccine 100 $\mu$L equivalent | - | - | Solution | Sublingual | 30 |
| Ex. 142 | Tetanus toxoid-conjugated Haemophilus influenzae type b polysaccharide | Vaccine 100 $\mu$L equivalent | Tretinoin | 100 | Solution | Sublingual | 30 |

(continued)

| | Antigen | | Nuclear receptor ligand | | Formulation | Administration route | Amount [$\mu$L] |
|---|---|---|---|---|---|---|---|
| | Name | Amount [$\mu$g] | Name | Amount [$\mu$g] | | | |
| Ex. 143 | Tetanus toxoid-conjugated Haemophilus influenzae type b polysaccharide | Vaccine 100 $\mu$L equivalent | Liothyronine | 100 | Solution | Sublingual | 30 |
| Ex. 144 | Tetanus toxoid-conjugated Haemophilus influenzae type b polysaccharide | Vaccine 100 $\mu$L equivalent | Tamoxifen | 100 | Solution | Sublingual | 30 |
| Comp. Ex. 43 | Recombinant HBs antigen protein | Vaccine 100 $\mu$L equivalent | - | - | Solution | Transnasal | 10 |
| Ex. 145 | Recombinant HBs antigen protein | Vaccine 100 $\mu$L equivalent | Tretinoin | 50 | Solution | Transnasal | 10 |
| Ex. 146 | Recombinant HBs antigen protein | Vaccine 100 $\mu$L equivalent | Liothyronine | 50 | Solution | Transnasal | 10 |
| Ex. 147 | Recombinant HBs antigen protein | Vaccine 100 $\mu$L equivalent | Tamoxifen | 10 | Solution | Transnasal | 10 |
| Comp. Ex. 44 | Recombinant HBs antigen protein | Vaccine 100 $\mu$L equivalent | - | - | Solution | Sublingual | 30 |
| Ex. 148 | Recombinant HBs antigen protein | Vaccine 100 $\mu$L equivalent | Tretinoin | 100 | Solution | Sublingual | 30 |
| Ex. 149 | Recombinant HBs antigen protein | Vaccine 100 $\mu$L equivalent | Liothyronine | 100 | Solution | Sublingual | 30 |
| Ex. 150 | Recombinant HBs antigen protein | Vaccine 100 $\mu$L equivalent | Tamoxifen | 100 | Solution | Sublingual | 30 |
| Comp. Ex. 45 | Live attenuated yellow fever virus | Vaccine 100 $\mu$L equivalent | - | - | Solution | Transnasal | 10 |
| Ex. 151 | Live attenuated yellow fever virus | Vaccine 100 $\mu$L equivalent | Tretinoin | 50 | Solution | Transnasal | 10 |
| Ex. 152 | Live attenuated yellow fever virus | Vaccine 100 $\mu$L equivalent | Liothyronine | 50 | Solution | Transnasal | 10 |
| Ex. 153 | Live attenuated yellow fever virus | Vaccine 100 $\mu$L equivalent | Tamoxifen | 10 | Solution | Transnasal | 10 |

(continued)

| | Antigen | | Nuclear receptor ligand | | Formulation | Administration route | Amount [μL] |
|---|---|---|---|---|---|---|---|
| | Name | Amount [μg] | Name | Amount [μg] | | | |
| Comp. Ex. 46 | Live attenuated yellow fever virus | Vaccine 100 μL equivalent | - | - | Solution | Sublingual | 30 |
| Ex. 154 | Live attenuated yellow fever virus | Vaccine 100 μL equivalent | Tretinoin | 100 | Solution | Sublingual | 30 |
| Ex. 155 | Live attenuated yellow fever virus | Vaccine 100 μL equivalent | Liothyronine | 100 | Solution | Sublingual | 30 |
| Ex. 156 | Live attenuated yellow fever virus | Vaccine 100 μL equivalent | Tamoxifen | 100 | Solution | Sublingual | 30 |

[Table 12]

| | Antigen | | Nuclear receptor ligand | | Formulation | Administration route | Amount [μL] |
|---|---|---|---|---|---|---|---|
| | Name | Amount [μg] | Name | Amount [μg] | | | |
| Comp. Ex. 47 | Tetanus toxoid | Vaccine 100 μL equivalent | - | - | Solution | Transnasal | 10 |
| Ex. 157 | Tetanus toxoid | Vaccine 100 μL equivalent | Tretinoin | 50 | Solution | Transnasal | 10 |
| Ex. 158 | Tetanus toxoid | Vaccine 100 μL equivalent | Liothyronine | 50 | Solution | Transnasal | 10 |
| Ex. 159 | Tetanus toxoid | Vaccine 100 μL equivalent | Tamoxifen | 10 | Solution | Transnasal | 10 |
| Comp. Ex. 48 | Tetanus toxoid | Vaccine 100 μL equivalent | - | - | Solution | Sublingual | 30 |
| Ex. 160 | Tetanus toxoid | Vaccine 100 μL equivalent | Tretinoin | 100 | Solution | Sublingual | 30 |
| Ex. 161 | Tetanus toxoid | Vaccine 100 μL equivalent | Liothyronine | 100 | Solution | Sublingual | 30 |
| Ex. 162 | Tetanus toxoid | Vaccine 100 μL equivalent | Tamoxifen | 100 | Solution | Sublingual | 30 |
| Comp. Ex. 49 | Live attenuated varicella virus | Vaccine 100 μL equivalent | - | - | Solution | Transnasal | 10 |
| Ex. 163 | Live attenuated varicella virus | Vaccine 100 μL equivalent | Tretinoin | 50 | Solution | Transnasal | 10 |
| Ex. 164 | Live attenuated varicella virus | Vaccine 100 μL equivalent | Liothyronine | 50 | Solution | Transnasal | 10 |
| Ex. 165 | Live attenuated varicella virus | Vaccine 100 μL equivalent | Tamoxifen | 10 | Solution | Transnasal | 10 |
| Comp. Ex. 50 | Live attenuated varicella virus | Vaccine 100 μL equivalent | - | - | Solution | Sublingual | 30 |
| Ex. 166 | Live attenuated varicella virus | Vaccine 100 μL equivalent | Tretinoin | 100 | Solution | Sublingual | 30 |
| Ex. 167 | Live attenuated varicella virus | Vaccine 100 μL equivalent | Liothyronine | 100 | Solution | Sublingual | 30 |

(continued)

|  | | Antigen | | Nuclear receptor ligand | | Formulation | Administration route | Amount [µL] |
| --- | --- | --- | --- | --- | --- | --- | --- | --- |
|  | | Name | Amount [µg] | Name | Amount [µg] | | | |
| Ex. 168 | | Live attenuated varicella virus | Vaccine 100 µL equivalent | Tamoxifen | 100 | Solution | Sublingual | 30 |
| Comp. Ex. 51 | | Live BCG | Vaccine 30 µL equivalent | - | - | Solution | Transnasal | 10 |
| Ex. 169 | | Live BCG | Vaccine 30 µL equivalent | Tretinoin | 50 | Solution | Transnasal | 10 |
| Ex. 170 | | Live BCG | Vaccine 30 µL equivalent | Liothyronine | 50 | Solution | Transnasal | 10 |
| Ex. 171 | | Live BCG | Vaccine 30 µL equivalent | Tamoxifen | 10 | Solution | Transnasal | 10 |
| Comp. Ex. 52 | | Live BCG | Vaccine 30 µL equivalent | - | - | Solution | Sublingual | 30 |
| Ex. 172 | | Live BCG | Vaccine 30 µL equivalent | Tretinoin | 100 | Solution | Sublingual | 30 |
| Ex. 173 | | Live BCG | Vaccine 30 µL equivalent | Liothyronine | 100 | Solution | Sublingual | 30 |
| Ex. 174 | | Live BCG | Vaccine 30 µL equivalent | Tamoxifen | 100 | Solution | Sublingual | 30 |
| Comp. Ex. 53 | | Inactivated rabies virus | Vaccine 200 µL equivalent | - | - | Solution | Transnasal | 10 |
| Ex. 175 | | Inactivated rabies virus | Vaccine 200 µL equivalent | Tretinoin | 50 | Solution | Transnasal | 10 |
| Ex. 176 | | Inactivated rabies virus | Vaccine 200 µL equivalent | Liothyronine | 50 | Solution | Transnasal | 10 |
| Ex. 177 | | Inactivated rabies virus | Vaccine 200 µL equivalent | Tamoxifen | 10 | Solution | Transnasal | 10 |
| Comp. Ex. 54 | | Inactivated rabies virus | Vaccine 200 µL equivalent | - | - | Solution | Sublingual | 30 |
| Ex. 178 | | Inactivated rabies virus | Vaccine 200 µL equivalent | Tretinoin | 100 | Solution | Sublingual | 30 |

(continued)

| | Antigen | | Nuclear receptor ligand | | Formulation | Administration route | Amount [$\mu$L] |
|---|---|---|---|---|---|---|---|
| | Name | Amount [$\mu$g] | Name | Amount [$\mu$g] | | | |
| Ex. 179 | Inactivated rabies virus | Vaccine 200 $\mu$L equivalent | Liothyronine | 100 | Solution | Sublingual | 30 |
| Ex. 180 | Inactivated rabies virus | Vaccine 200 $\mu$L equivalent | Tamoxifen | 100 | Solution | Sublingual | 30 |

(Examples 181 to 184, Comparative Example 55)

A cream for transdermal administration was prepared according to the formulation shown in Table 13 in the same manner as the cream for transdermal administration of Table 7. The right back of a mouse (C57BL6 NCr mouse, female, 7 weeks old) was shaved, and after the skin was subjected to a corneum removing treatment five times with an OPP tape (EZ Dunplon No. 3301EZ, Nitto Denko Corporation), the cream was administered to the skin (minimally invasive administration), and the left back was shaved at the same time. Twenty-four hours later, the cream for transdermal administration on the right back was removed. One week after the administration, the skin of the left back of the mouse was subjected to a corneum removing treatment in the same manner as above, and the cream for transdermal administration was administered thereto. The cream was removed 24 hours later. One week after the second administration, the mouse serum was taken, and the antigen (OVA)-specific IgG antibody in the serum was determined by ELISA. Also in this immunization using the minimally invasive administration, humoral immunity specific to the administered antigen can be induced.

[Table 13]

| No. | Administration route | Dosage form | Antigen | | Nuclear receptor ligand | | Pharmacological effect | Immunological evaluation mouse |
|---|---|---|---|---|---|---|---|---|
| | | | Name | Amount [Parts by weight] | Name | Amount [Parts by weight] | | |
| Comp. Ex. 55 | Transdermal (minimally invasive) | Cream | OVA | 5 | - | - | - | C57BL6 |
| Ex. 181 | Transdermal (minimally invasive) | Cream | OVA | 5 | Tretinoin | 5 | RAR activation | C57BL6 |
| Ex. 182 | Transdermal (minimally invasive) | Cream | OVA | 5 | Levothyroxine sodium | 5 | TR activation | C57BL6 |
| Ex. 183 | Transdermal (minimally invasive) | Cream | OVA | 5 | Liothyronine | 5 | TR activation | C57BL6 |
| Ex. 184 | Transdermal (minimally invasive) | Cream | OVA | 5 | Raloxifene hydrochloride | 5 | ER modulation | C57BL6 |

[Table 14]

| Additve | Amount [Parts by weight] |
|---|---|
| White Vaseline | 60.7 |
| Sorbitan monostearate | 0.7 |
| Isostearic acid | 12 |
| Benzyl alcohol | 2.4 |
| Cetanol | 2.4 |
| Stearyl alcohol | 3.5 |
| Polysorbate 60 | 3.5 |
| Concentrated glycerin | 2.4 |
| Purified water | 12.4 |
| Total | 100 |

INDUSTRIAL APPLICABILITY

The composition for promoting immunity induction and vaccine pharmaceutical composition of the present invention are universally usable for inducing immunity to various antigens and suitable not only for subcutaneous administration but also for transdermal administration or transmucosal administration.

**Claims**

1. A composition for promoting immunity induction, comprising:

   a nuclear receptor ligand.

**2.** The composition for promoting immunity induction according to claim 1,
wherein the nuclear receptor ligand is at least one selected from the group consisting of a retinoid receptor agonist, a retinoid X receptor agonist, a thyroid hormone receptor agonist, and an estrogen receptor modulator.

**3.** The composition for promoting immunity induction according to claim 1,
wherein the nuclear receptor ligand is at least one selected from the group consisting of a retinoid receptor agonist, a thyroid hormone receptor agonist, and an estrogen receptor modulator and is for inducing humoral immunity.

**4.** The composition for promoting immunity induction according to claim 1,
wherein the nuclear receptor ligand is at least one of a retinoid receptor agonist or a retinoid X receptor agonist and is for inducing cellular immunity.

**5.** The composition for promoting immunity induction according to claim 1, 2, 3, or 4, further comprising a helper peptide.

**6.** A vaccine pharmaceutical composition, comprising:

an antigen for inducing immunity; and
the composition for promoting immunity induction according to claim 1, 2, 3, 4, or 5.

**7.** The vaccine pharmaceutical composition according to claim 6, which is administered to a body surface.

**8.** The vaccine pharmaceutical composition according to claim 6, which is administered by intradermal injection, subcutaneous injection, or intramuscular injection.

## FIG.1

## FIG.2

# FIG.3

# FIG.4

## FIG.5

## FIG.6

FIG.7

## INTERNATIONAL SEARCH REPORT

| International application No. |
| --- |
| PCT/JP2015/072103 |

**A. CLASSIFICATION OF SUBJECT MATTER**
*A61K39/39*(2006.01)i, *A61K45/00*(2006.01)i, *A61P37/02*(2006.01)i, *A61P43/00*(2006.01)i

According to International Patent Classification (IPC) or to both national classification and IPC

**B. FIELDS SEARCHED**

Minimum documentation searched (classification system followed by classification symbols)
A61K39/39, A61K45/00, A61P37/02, A61P43/00

Documentation searched other than minimum documentation to the extent that such documents are included in the fields searched
Jitsuyo Shinan Koho      1922–1996    Jitsuyo Shinan Toroku Koho   1996–2015
Kokai Jitsuyo Shinan Koho   1971–2015    Toroku Jitsuyo Shinan Koho   1994–2015

Electronic data base consulted during the international search (name of data base and, where practicable, search terms used)
JSTPlus/JMEDPlus/JST7580(JDreamIII), CAplus/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | SEMBA R.D., Vitamin A,immunity,and infection., Clinical Infectious Diseases, 1994, 19(3), p.489-499 particularly, page 492, left column, last paragraph to right column, 1st paragraph | 1,2<br>5-8 |
| X<br>Y | ALLENDE L.M.et al, Retinol (vitamin A) is a cofactor in CD3-induced human T-lymphocyte activation., Immunology, 1997, 90, p.388-396 particularly, SUMMARY, page 393, left column, last paragraph to right column, 1st paragraph | 1,2<br>5-8 |
| X<br>Y | Makoto IWATA et al., "Retinoic Acid wa Juyotai RAR o Kaishite T Saibo ni Chokusetsu Sayo shi Th1, Th2 eno Bunka o Seigyo suru", Proceedings of the Japanese Society for Immunology, 2003, 33, page 209, 2-H-W27-3-P, particularly, column of [Kekka to Kosatsu] | 1-3<br>5-8 |

| ☒ Further documents are listed in the continuation of Box C. | ☐ See patent family annex. |
| --- | --- |

| *   Special categories of cited documents: | "T"   later document published after the international filing date or priority date and not in conflict with the application but cited to understand the principle or theory underlying the invention |
| --- | --- |
| "A"   document defining the general state of the art which is not considered   to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 08 October 2015 (08.10.15) | 20 October 2015 (20.10.15) |

| Name and mailing address of the ISA/<br>Japan Patent Office<br>3-4-3,Kasumigaseki,Chiyoda-ku,<br>Tokyo 100-8915,Japan | Authorized officer |
| --- | --- |
| | Telephone No. |

Form PCT/ISA/210 (second sheet) (July 2009)

53

**INTERNATIONAL SEARCH REPORT**

| International application No. |
|---|
| PCT/JP2015/072103 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | STEPHENSEN,C.B. et al, Vitamin A enhances in vitro Th2 development via retinoid X receptor Pathway., J. Immunol., 2002, 168(9), p.4495-4503 particularly, abstract | 1-3<br>5-8 |
| X<br>Y | LESSARD M. et al, Cell-mediated and humoral immune responses in broiler chickens maintained on diets containing different levels of vitamin A., Poult. Sci., 1997, 76(10), p.1368-1378 particularly, ABSTRACT | 1,2,4<br>5-8 |
| X<br>Y | YOSHINO S. et al, Prenatal exposure to bisphenol A up-regulates immune responses, including T helper 1 and T helper 2 responses, in mice., Immunology, 2004, 112(3), p.489-495 particularly, SUMMARY | 1,2<br>5-8 |
| X<br>Y | VERTHELYI D., Sex hormones as immunomodulators in health and disease., Int. Immunopharmacol., 2001, 1(6), p.983-993 particularly, Abstract | 1-3<br>5-8 |
| X<br>Y | Tianyi LIU et al., "Estrogen ga DC o Kaishite Th Otosei ni Oyobosu Eikyo no Hyoka", Japanese Journal of Allergology, 2005, 54(8/9), page 1092, 341, particularly, column of [Kekka] | 1-3<br>5-8 |
| X<br>Y | Sho MATSUSHITA, "Analysis of glycolipid antigens associated with food allergy", Urakami Foundation Memories, 2004, 12, pages 43 to 50, particularly, page 45, right column, lines 3 to 5 | 1-3<br>5-8 |
| X<br>Y | Masaya TAKAMOTO, "Kankyo Hormone no Th1/Th2 Balance eno Eikyo to Kiseichu Kansen", Allergy-Ka, 2003, 16(5), pages 412 to 418, particularly, page 414, right column, line 1 to page 416, left column, line 9, page 416, left column, line 11 to right column, line 6, right column, 8th line from the bottom to 6th line from the bottom | 1-3<br>5-8 |
| Y | JP 2013-530157 A (Selecta Biosciences, Inc.), 25 July 2013 (25.07.2013), particularly, example 5<br>& JP 2013-526617 A    & JP 2013-528181 A<br>& JP 2013-530158 A    & US 2011/0293700 A1<br>especially, paragraph [0222]<br>& WO 2011/150240 A1    & EP 2575773 A<br>& AU 2011258147 A    & CA 2798493 A<br>& CN 102905728 A    & CN 103118700 A<br>& KR 10-2013-0108983 A | 5-8 |

Form PCT/ISA/210 (continuation of second sheet) (July 2009)

**EP 3 189 853 A1**

<table>
<tr><td colspan="2" align="center"><strong>INTERNATIONAL SEARCH REPORT</strong></td><td>International application No.<br>PCT/JP2015/072103</td></tr>
</table>

C (Continuation). DOCUMENTS CONSIDERED TO BE RELEVANT

| Category* | Citation of document, with indication, where appropriate, of the relevant passages | Relevant to claim No. |
|---|---|---|
| A | DEPAOLO R.W. et al, Co-adjuvant effects of retinoic acid and IL-15 induce inflammatory immunity to dietary antigens., Nature, 2011, 471(7337), p.220-224 | 1-8 |
| A | MORA J.R. et al, Generation of gut-homing IgA-secreting B cells by intestinal dendritic cells., Science, 2006, 314(5802), p.1157-1160 | 1-8 |
| A | KATO T. et al, Endocrine disruptors that deplete glutathione levels in APC promote Th2 polarization in mice leading to the exacerbation of airway inflammation., Eur. J. Immunol., 2006, 36(5), p.1199-1209 | 1-8 |
| A | Tadasu FURUSHO et al., "Saibosei Men'eki Kino no Hendo ni Oyobosu Vitamin A Oyobi Carotenoid Toyo no Eikyo", Vitamins, 2001, 75(4), page 220, 2-I-3 | 1-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIPSCOMB MF. et al.** *Physiol Rev.,* 2002, vol. 82, 97-130
- **ZHOU L. et al.** *Immunity,* 2009, vol. 30, 646-655
- **MAZZONI A. et al.** *J Leukoc Biol.,* 2004, vol. 75, 721-730
- **STEVCEVA L. et al.** *Curr Pharm Des,* 2005, vol. 11, 801-811


SEQUENCE LISTING FREE TEXT

SEQUENCE LISTING

<110> NITTO DENKO CORPORATION

<120> Immune induction promoting composition comprising intranuclear receptor ligand, and Vaccine composition

<130> F6316WO

<150> JP2014-159000
<151> 2014-08-04

<160> 13

<170> PatentIn version 3.5

<210> 1
<211> 9
<212> PRT
<213> Homo sapiens

<400> 1

Ala Tyr Ala Cys Asn Thr Ser Thr Leu
1               5


<210> 2
<211> 9
<212> PRT
<213> Homo sapiens

<400> 2

Glu Tyr Ile Leu Ser Leu Glu Glu Leu
1               5


<210> 3
<211> 9
<212> PRT
<213> Homo sapiens

<400> 3

Thr Tyr Leu Pro Thr Asn Ala Ser Leu
1               5


<210> 4
<211> 9
<212> PRT
<213> Homo sapiens

<400> 4

Ile Met Pro Lys Ala Gly Leu Leu Ile
1               5


<210> 5
<211> 9
<212> PRT

<213> Homo sapiens

<400> 5

Val Leu Gln Glu Leu Asn Val Thr Val
1               5


<210> 6
<211> 9
<212> PRT
<213> Homo sapiens

<400> 6

Lys Val Phe Gly Ser Leu Ala Phe Val
1               5


<210> 7
<211> 9
<212> PRT
<213> Unknown

<220>
<223> MAGE3_A02 Peptide

<400> 7

Lys Val Ala Glu Ile Val His Phe Leu
1               5


<210> 8
<211> 9
<212> PRT
<213> Homo sapiens

<400> 8

Lys Ile Phe Gly Ser Leu Ala Phe Leu
1               5


<210> 9
<211> 9
<212> PRT
<213> Homo sapiens

<400> 9

Ser Thr Ala Pro Pro Val His Asn Val
1               5


<210> 10
<211> 9
<212> PRT
<213> hepatitis C virus

<400> 10

Asp Leu Met Gly Tyr Ile Pro Ala Val

1                    5


<210>  11
<211>  9
<212>  PRT
<213>  hepatitis B virus

<400>  11

Trp Leu Ser Leu Leu Val Pro Phe Val
1                    5


<210>  12
<211>  13
<212>  PRT
<213>  Artificial Sequence

<220>
<223>  PADRE


<220>
<221>  MOD_RES
<222>  (1)..(1)
<223>  D-alanine

<220>
<221>  MOD_RES
<222>  (3)..(3)
<223>  cyclohexylalanine

<220>
<221>  MOD_RES
<222>  (13)..(13)
<223>  D-alanine

<400>  12

Ala Lys Ala Val Ala Ala Trp Thr Leu Lys Ala Ala Ala
1                    5                    10


<210>  13
<211>  8
<212>  PRT
<213>  Gallus gallus

<400>  13

Ser Ile Ile Asn Phe Glu Lys Leu
1                    5